# FASCICULE DE BREVET EUROPEEN

(11) **EP 1 314 040 B1**
(45) Date de publication et mention de la délivrance du brevet: **09.04.2008**
(21) Numéro de dépôt: 01963064.9
(22) Date de dépôt: 07.08.2001
(51) Int. Cl.: G01N 33/68

(54) **UTILISATION DE LA PROTEINE GRF1 POUR LE CRIBLAGE DE MOLECULES**
VERWENDUNG DES PROTEINS GRF-1 ZUR AUSWAHL VON MOLEKÜLEN
USE OF GRF1 PROTEIN FOR SCREENING MOLECULES

(30) Priorité: 10.08.2000 FR 0010539
(43) Date de publication de la demande: 28.05.2003
(73) Titulaire: Aventis Pharma S.A., 92160 Antony (FR)
(72) Inventeur: ITIER, Jean-Michel, F-91360 VILLEMOISSON SUR ORGE (FR); MULTON, Marie-Christine, F-78000 VERSAILLES (FR); RET, Gwénaëlle, 91510 LARDY (FR); STUTZMANN, Jean-Marie, F-94440 VILLECRESNES (FR); WAHL, Florence, F-75004 PARIS (FR)
(74) Mandataire: Bouvet, Philippe
(86) Numéro de dépôt international: PCT/FR2001/002561
(87) Numéro de publication internationale: WO 2002/012901

(56) Documents cités:
- WO-A-00/40718
- WO-A-00/43510
- WO-A-93/21314

## Description

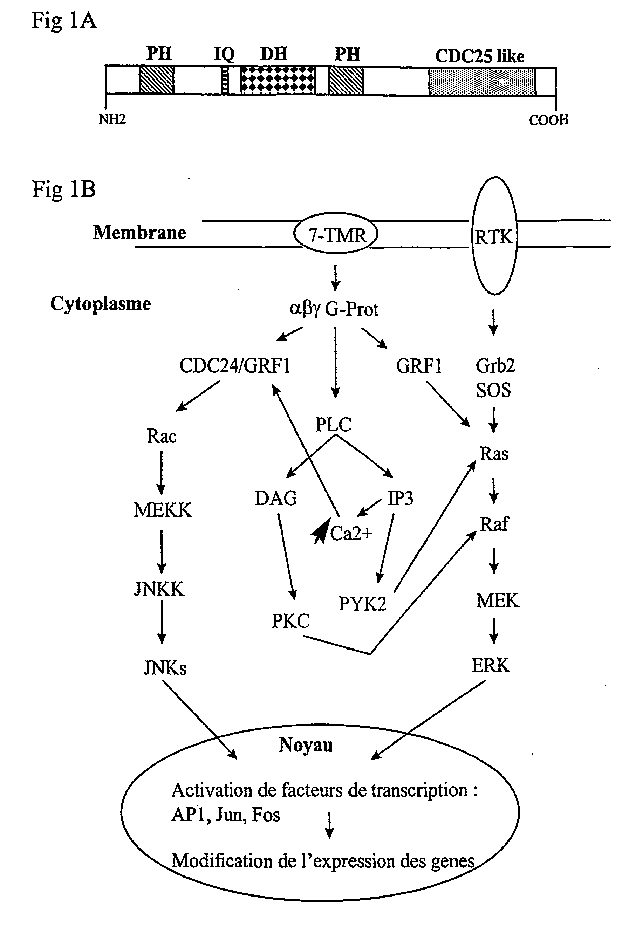

La présente invention a trait au domaine de la biologie et de la signalisation cellulaire dans les neurones. Plus précisément, la présente invention concerne de nouvelles utilisations de tout ou partie de la protéine GRF1 (Guanine Nucleotide Releasing Factor 1) pour le criblage de molécules présentant une activité de protection contre la mort neuronale et de traitement de l'obésité. La protéine GRF1, qui est représentée schématiquement à la Figure 1A, a été originellement découverte chez l'homme pour sa capacité à moduler l'état d'activation de la protéine p21Ras. Les séquences des protéines GRF1 humaine, de souris et de rat sont respectivement les séquences SEQ ID N°1, SEQ ID N°2 et SEQ ID N°3. La demande WO 93/21314 et le brevet US 5,656,595 décrivent l'identification, l'isolement ainsi que la caractérisation de la forme humaine de cette protéine.

WO0040718 décrit des mutants en positions 1184 et 1124 de la protéine GNRF de souris. Selon cette demande ces protéines ou des acides nucléiques les codant pourraient être utilisées à des fins thérapeutiques, en particulier dans le traitement des tumeurs.

GRF1 possède plusieurs domaines fonctionnels dits domaines PH, IQ, DH, et CDC25 dont la signification et l'implication sont précisées ci-après.

La localisation des domaines fonctionnels de GRF1 sur les séquences protéiques humaine, de souris et de rat est indiquée dans le tableau ci après.

GRF1 comporte un domaine DH (Db1 homology) qui présente une forte homologie de séquence avec une région du proto-oncogène Dbl. Dbl est un facteur d'échange des petites protéines G de la famille Rho/Rac/Cdc42Hs impliquées dans la régulation de divers signaux cellulaires contrôlant soit la motilité du cytosquelette, soit l'activation des kinases dites de "stress" (1).

Dans la protéine Dbl, l'activité d'échange du GDP/GTP sur les protéines de la famille Rho est portée par le domaine DH. Celui-ci est retrouvé dans d'autres facteurs d'échange des protéines G de petit poids moléculaire dont les protéines GRFs et les protéines SOSs. Des résultats ont été publiés qui suggèrent que le domaine DH de GRF1 est fonctionnel sur Rac (activation de Rac sous forme GTP) et conduit à l'activation de la JNK1 dans des cellules en culture qui surexpriment la sous unité béta/gamma des protéines G hétéro-trimériques (2). Par ailleurs, le rôle fonctionnel du domaine DH de GRF1 dans des processus neuronaux a également été suggéré par les résultats d'expériences de mutagenèse dirigée qui montrent que l'activation de la protéine GRF1 par le calcium requiert un domaine DH intègre (3, 4).

Le calcium joue un rôle important dans la régulation des activités neuronales : sécrétion de neuromédiateurs, croissance des neurones, mort/survie cellulaire, adaptation et stimulation de la transmission synaptique, activation transcriptionnelle de certains gènes. Des études réalisées sur des cultures primaires de neurones de cortex de rats nouveaux nés ont montré que l'activation de Ras par GRF1 était augmentée en présence de calcium. Cette activation est régulée par la fixation de la calmoduline sur le domaine IQ de GRF1 (3-5).

L'activité d'échange de GRF1 pour les protéines Ras (Ha, Ki et N-Ras) est quant à elle portée par la région homologue à CDC25 de *Saccharomyces cerevisiae*, située à l'extrémité carboxy-terminale de la molécule (6).

GRF1 possède deux domaines PH (Pleckstrin Homology) qui sont des domaines d'interaction entre protéines et qui sont vraisemblablement impliqués dans la liaison aux sous-unités béta-gamma des protéines G hétéro-trimériques (7-9).

On connaît aujourd'hui plus en détail les fonctions de la protéine GRF1, découverte initialement pour son activité d'échange sur le proto-oncogène Ras. Contrairement aux protéines SOS1, SOS2 et GRF2, trois autres facteurs d'échange de Ras qui sont exprimés de façon ubiquitaire, il est maintenant admis que l'expression du gène *grf1* est restreinte au système nerveux central chez l'homme, la souris et le rat ( 6, 10, 11).

Dans le cerveau, la régionalisation de cette expression a été finement étudiée. Chez le rat, elle est importante dans l'hippocampe, le néocortex, quelques noyaux profonds et les cellules en grain du lobe antérieur du cervelet (12). Chez la souris, elle a également été décrite comme étant abondante dans l'amygdale (13) et l'hypothalamus (14).

Parmi les différents types cellulaires présents dans le système nerveux central, l'expression du gène *grf1* semble restreinte aux neurones (15). Ce gène subit dans le temps, un contrôle transcriptionnel strict puisque qu'il n'est pas exprimé pendant la vie embryonnaire et ne commence à être transcrit qu'au moment de la naissance pour atteindre un niveau maximum autour du quinzième jour (14, 15).

L'expression de *grf1* est contrôlée par un mécanisme transcriptionnel particulier appelé empreinte parentale qui se traduit chez la souris par une expression du gène à partir de l'allèle d'origine paternel tandis que l'allèle maternel est silencieux (14, 16). Il est intéressant de noter que certains gènes (proto-oncogènes) normalement soumis à empreinte parentale sont impliqués dans la prolifération cellulaire et l'apparition de tumeurs lorsqu'il y a perte du mécanisme d'empreinte et que les deux allèles sont transcrits.

Alors que la voie d'activation de Ras par les facteurs d'échange SOS est bien documentée, il apparaît que l'on sait très peu de choses sur les voies de signalisation qui utilisent GRF1 et sur sa fonction biologique *in vivo*.

Il semble que, contrairement aux autres facteurs d'échange de Ras de la famille SOS, GRF1 ne transduit pas les signaux résultant de la fixation de ligands sur des récepteurs à activité tyrosine kinase, mais plutôt ceux issus de récepteurs à sept domaines transmembranaires couplés aux protéines G hétéro-trimériques (17-19).

Enfin on notera que certaines des fonctions biologiques de GRF1 ont pu être abordées *in vivo* grâce à l'étude du phénotype de souris porteuses d'une mutation inactivante du gène *grf1* - souris knock-out ou KO, obtenues par la technique de recombinaison homologue - qui n'expriment plus GRF1 (13, 14). Ces animaux sont peu performants, voir déficients dans des tests d'apprentissage et de mémorisation qui mettent en oeuvre des stimuli "aversifs". Selon les auteurs de ce travail, l'intégrité de la région du cerveau appelée amygdale serait altérée chez les souris mutantes et responsable du phénotype observé (13). Néanmoins aucune indication n'est donnée quant à une possible utilisation de ces souris pour le criblage de molécules présentant une activité thérapeutique.

Les atteintes physiques du cerveau telles que l'ischémie cérébrale ou le trauma cérébral (atteintes aiguës ou accidentelles) et les maladies neurodégénératives telles que la maladie d'Alzheimer et la maladie de Parkinson, restent une des causes premières de mortalité et de morbidité dans les pays développés.

Il a été montré que l'apoptose constituait l'un des mécanismes conduisant à ces atteintes.

Un nombre considérable de molécules agissant sur divers événements physiopathologiques causés par des atteintes au système nerveux central a été évalué avec succès en recherche expérimentale dans différents modèles et espèces. Bien que certaines d'entre elles ont été ou sont utilisées dans des essais cliniques pour ces désordres, aucune des molécules testées n'a montré de réelle efficacité lors des études en phase III ou lors de leur mise sur le marché.

La seule médication actuellement utilisée pour le traitement de l'ischémie cérébrale consiste en l'injection de thrombolytiques aux seuls patients ne présentant pas de risques hémorragiques.

La demanderesse a montré, par l'étude de souris portant une mutation inactivante du gène *grf1* que l'absence d'expression de la protéine GRF1 conférait une protection contre l'atteinte physique du cerveau lors d'une ischémie cérébrale.

Par ailleurs en quelques dizaines d'années, l'obésité est devenue un problème majeur de santé publique dans les pays industrialisés où elle touche maintenant 20 à 30 % de la population. Ces chiffres devraient encore croître de façon alarmante dans les années à venir. Du fait de ses origines multifactorielles qui prennent leurs sources à des degrés plus ou moins importants parmi des facteurs environnementaux d'une part (comportements alimentaires, accès à la nourriture, dépense énergétique,...) et des origines génétiques multiples d'autre part, l'obésité constitue un véritable défi pour la médecine.

La physiopathologie des maladies liées au poids, est complexe et hétérogène. L'obésité est fréquemment associée à un risque accru de décès, à des maladies cardio-vasculaires, à un diabète non-insulinodépendant et à une résistance à l'insuline. Des troubles psychologiques et comportementaux comme l'anxiété et la dépression viennent encore s'ajouter au tableau clinique de la maladie. Par ailleurs et phénotypiquement à l'opposé, la perte excessive de poids dans des contextes environnementaux (malnutrition) ou pathologiques (anorexie mentale par exemple) particuliers est associée à une morbidité et une mortalité importante. Pour ces différentes raisons, l'identification d'un gène jouant un rôle dans la prise de poids, présente un intérêt considérable que l'on s'intéresse au traitement de l'obésité ou à celui de la perte de poids excessive.

La demanderesse a montré, par l'étude de souris portant une mutation inactivante du gène *grf1* que l'expression de la protéine GRF1 est indispensable à la régulation de l'expression de la leptine. La leptine est une cytokine associée à la sensation de satiété qui joue un rôle majeur dans le contrôle de la prise de poids (20).

Dans le contexte scientifique tel qu'il vient d'être résumé, il existe encore le besoin d'une meilleure compréhension des différents mécanismes en jeu pouvant conduire notamment à des désordres impliquant une apoptose neuronale ou liés au métabolisme de la leptine.

Il existe en outre un besoin de médicaments efficaces dans le traitement de désordres du système nerveux central et dans le traitement de l'obésité.

La Demanderesse s'est attachée à rechercher des composés destinés à la prévention et/ou au traitement de désordres touchant notamment au système nerveux central et à l'obésité.

Elle a montré, à l'aide de souris porteuses d'une mutation inactivante du gène *grf1* comme modèle d'étude, que GRF1 intervient dans ces désordres.

La présente invention est donc relative à l'utilisation d'une protéine présentant une identité d'au moins 85% avec une GRF1 ou de cellules exprimant une protéine présentant une identité d'au moins 85% avec une GRF1, dans des procédés de détection de composés destinés à la prévention et / ou au traitement de pathologies ou de désordres du système nerveux central impliquant une mort neuronale, telle que l'apoptose.

Elle est en outre relative à des composés destinés à la prévention et / ou au traitement de pathologies ou de désordres du système nerveux central impliquant une mort neuronale ou liés à l'obésité.

La protéine GRF1 est préférentiellement d'origine humaine. Elle peut être néanmoins de toute autre origine et en particulier être la protéine GRF1 de souris ou de tout autre mammifère. Elle peut aussi être toute protéine présentant un identité d'au moins 85% et préférentiellement 90% avec une protéine GRF1, et en particulier avec la protéine GRF1 humaine présentant la séquence SEQ ID N°1, ou avec une protéine GRF1 d'origine animale telle que celles présentant les séquences SEQ ID N°2 ou SEQ ID N°3 . On entend par partie de la protéine GRF1 une séquence d'acides aminés comprenant une partie fonctionnelle de la protéine GRF1, et en particulier les séquences correspondant à tout ou partie d'un des domaines PHs, DH, ou CDC25 des protéines GRF 1.

L'un des avantages des procédés de criblage objets de la présente invention réside en particulier dans la position de GRF1 en amont des JNKs (Figure 1B) ce qui présente comme avantage de pouvoir réguler de façon spécifique les voies d'activation des JNKs qui passeraient par GRF1.

Un autre avantage réside dans la mise en évidence de l'absence de toxicité de ces composés , les souris inactivées pour *grf1* étant viables et en bonne santé.

Le procédé de criblage selon la présente invention utilisant tout ou partie de la protéine GRF1 peut comprendre des étapes de mesure de :
- la phosphorylation de la protéine GRF1 ; ou de
- l'activité d'échange de la protéine GRF1 sur soit des protéines de la famille Ras (Ha, K, N-Ras), soit les protéines Rac et Cdc42; de façon directe ou indirecte, ou de
- l'interaction entre la protéine GRF1 et soit les sous-unités béta-gamma des protéines G hétérotrimériques, soit les petites protéines G de la famille Ras, soit Rac et Cdc42.
- la transformation cellulaire par GRF 1

Ainsi selon un premier mode de mise en oeuvre avantageux la présente invention est relative à un procédé de criblage ou de détection de composés destinés à la prévention et / ou au traitement de pathologies du système nerveux central impliquant une mort neuronale comprenant les étapes consistant à :
(i) mettre en culture des cellules exprimant la protéine GRF1 en présence d'un composé phosphorylé marqué et d'un composé à tester,
(ii) lyser lesdites cellules, et
(iii) mesurer la quantité de protéine GRF 1 marquée.

Le lysat cellulaire obtenu à l'étape (ii) peut être mis en incubation dans des puits de plaques de microtitration préalablement recouverts d'un anticorps anti-GRF1.

La phosporylation de GRF1 peut aussi être mesurée à l'aide d'un anticorps spécifique d'un acide aminé phosphorylé.

Ainsi, selon un autre mode de mise en oeuvre avantageux, la présente invention est relative à un procédé de criblage ou de détection de composés destinés à la prévention et / ou au traitement de pathologies du système nerveux central impliquant une mort neuronale comprenant les étapes consistant à :
(i) mettre en culture des cellules exprimant la protéine GRF1 en présence d'un composé à tester,
(ii) lyser lesdites cellules
(iii) mesurer la quantité de GRF1 phosphorylée.

La mesure de la quantité de GRF1 phosphorylée est effectuée préférentiellement à l'aide d'un anticorps spécifique d'un acide aminé phosphorylé.

Lesdites cellules peuvent être mises en culture dans un milieu contenant de l'orthophosphate marqué au P³² ou au P³³.

La phosphorylation de la protéine GRF1 peut être réalisée dans lesdites cellules neuronales par addition de carbachol dans le milieu de culture ou bien dans des lignées cellulaires carencées en sérum pendant une nuit puis remises en incubation en présence de sérum, ou encore dans des lignées cellulaires cotransfectées par des ADNc codant pour les sous-unités béta-gamma des protéines G trimériques β1γ2, ou β1γ5, puis carencées en sérum pendant une nuit puis remises en incubation en présence de sérum.

Selon un autre mode de réalisation de l'invention le criblage comprend la mesure de l'activité d'échange de GRF1 sur certaines protéines de la famille des petites protéines G (Ras, Rac, Cdc42). Ainsi selon encore un autre mode de mise en oeuvre avantageux la présente invention est relative à un procédé de criblage ou de détection de composés destinés à la prévention et / ou au traitement de pathologies du système nerveux central impliquant une mort neuronale comprenant les étapes consistant à :
(i) mettre en présence tout ou partie de la protéine GRF1, une protéine de la famille des petites protéines G chargée en GDP ou en GTP marqué, et un composé à tester,
(ii) ajouter respectivement du GTP ou du GDP non marqué
(iii) mesurer la quantité de protéine G marquée.

Une telle protéine de la famille des petites protéines G peut être notamment Ras (53, 54, 55), Rac (56, 57, 58) ou Cdc42 (59).

La mesure de l'activité d'échange de la protéine GRF1 sur des protéines de la famille Ras, ou les protéines Rac et CDC42 peut être réalisée in vitro dans un milieu acellulaire par incubation dans des puits de plaques de microtitration d'un mélange réactionnel comprenant lesdites petites protéines G à l'état recombinant chargées en GDP tritié, GTP froid et tout ou partie de la protéine GRF1 à l'état recombinant. Une fraction aliquote dudit mélange réactionnel est prélevée et filtrée sur une membrane et où on mesure la radioactivité qui y est retenue, ou encore le contenu de chacun desdits puits est passé sur une colonne PD 10 et où on mesure la radioactivité de l'éluat.

Lesdites petites protéines G à l'état recombinant peuvent être choisies dans le groupe constitué par les protéines Ras, Cdc42 ou Rac sauvages soit sous forme de protéines de fusion et exprimées dans *E. coli* ou dans des cellules de mammifères, soit sous une forme étiquetée (tagged en anglais) dans un baculovirus, exprimées dans des cellules d'insectes et purifiées. La quantité de nucléotides échangés peut aussi être mise en évidence par rétention.

Avantageusement on ajoute des protéines de fusion GST-Raf (domaine RBD) ou GST-PAK (domaine CRIB), et un mélange d'IgG anti-GST et de protéine A PVT SPA (Scintillation Proximity Assay) couplée à des microsphères (Amersham) et en ce qu'on mesure la fluorescence desdites plaques de microtitration après centrifugation. Les formes liées au GDP ne permettent pas l'interaction. Les séquences de PAK et de Raf ont été publiées (voir respectivement les références 60 à 64, et 65 à 69).

On incube la protéine recombinante Ras (ou Rac) avec du GDP froid. Il se forme du Ras-GDP (ou Rac-GDP). Puis on ajoute du GTP tritié et GRF1. La réaction d'échange à lieu. Il se forme du Ras-GTP tritié. Au bout de 60 minutes on ajoute une protéine de fusion GST-Raf (ou GST-PAK si on travaille avec Rac). La protéine de fusion interagit avec Ras-GTP dont la quantité dans le milieu est dépendante de l'activité d'échange de GRF1. On ajoute les microsphères qui sont couplées à un anticorps anti-GST. Le complexe GST-Raf/Ras-GTP tritié se fixe sur les sphères. Celle-ci ont la capacité de scintiller lorsqu'elles sont à proximité d'une source radioactive. Elles permettent alors de quantifier la proportion de Ras-GTP et donc l'activité d'échange de GRF1 dans le milieu.

Le procédé de criblage peut aussi être mis en oeuvre dans un système cellulaire.

Ainsi il peut être réalisé par mise en contact des composés à cribler et d'une levure transformée par *grf1* et dont les gènes CDC25 et /ou CDC24 ont été inactivés ou mutés. Avantageusement la membrane d'une telle levure est préalablement perméabilisée et /ou au moins un des gènes impliqués dans les mécanismes de détoxification a été inactivé.

Il peut aussi être simplement réalisé dans une levure *S. cerevisiae* transformée par *grf1*. On mesure alors la levée des désordres métaboliques crées par l'expression de *grf1*.

Le procédé de criblage peut être aussi un système dit de double-hybride d'interaction protéine-protéine comportant une première protéine hybride constituée par une protéine de fusion entre Ras, CDC42Hs ou Rac et un domaine d'interaction à l'ADN et une deuxième protéine hybride constituée par une protéine de fusion entre tout ou partie de GRF1 et un domaine transactivateur. Il peut aussi être un système dit de "double-hybride plus un" consistant à exprimer dans le noyau d'une même levure *S*. *cerevisiae* soit tout ou partie de GRF1, PAK1 (domaine CRIB) fusionnée avec un domaine transactivateur et soit Rac soit CDC42Hs fusionnées avec un domaine d'interaction à l'ADN, soit tout ou partie de GRF1, c-Raf1 (RBD) fusionnée avec un domaine transactivateur et Ras fusionnée avec un domaine d'interaction à l'ADN.

Enfin le criblage peut être un criblage cellulaire comprenant les étapes consistant à
(i) transfecter des cellules mammifères immortalisées par un vecteur exprimant un transgène *gsf1* (tout ou partie) et conférant auxdites cellules le phénotype transformé et une résistance à un agent de sélection
(ii) sélectionner lesdites cellules transfectées et exprimant le transgène et les cloner dans un support de croissance en agar, la transformation par GRF1 permettant aux cellules de croître sans adhérer
(iii) ajouter lesdits composés à tester à une suspension desdites cellules et
(iv) détecter lesdits composés inhibiteurs de GRF1 par diminution du nombre de clones obtenus sur le milieu de croissance.

Comme il a déjà été mentionné, l'objet de la présente demande est la recherche de composés destinés à la fabrication de médicaments pour la prévention et / ou le traitement de diverses pathologies du système nerveux central (A) et de l'obésité (B). Du fait du rôle central de GRF1 les procédés objets de la présente demande peuvent aussi être utilisés pour cribler des molécules présentant une activité à l'encontre de certaines maladies cardio-vasculaires (B), les processus angiogéniques pathologiques (C), ainsi que vis à vis d'autres cibles biologiques (D).

### A- Système nerveux central

### 1-Maladies neurodégénératives aiguës

La présente invention permet le criblage de composés antagonisant l'effet de l'activité de GRF 1 et conférant une protection contre la mort neuronale induite par l'ischémie cérébrale, le traumatisme crânien ou cérébral et le traumatisme spinal ou médullaire.

### 2-Mort neuronale et apoptose

Les procédés objets de la présente invention peuvent être utilisés pour cribler des molécules pour le traitement ou la prévention de la neurodégénérescence impliquant l'apoptose neuronale, de la maladie de Parkinson, de la maladie d'Alzheimer, de la démence sénile, de la chorée de Huntington, de la sclérose latérale amyotrophique, des épilepsies, de la sclérose en plaques, des désordres cérébelleux et spinocérébelleux, des désordres cognitifs, du trauma crânien, du trauma médullaire, des traumatismes de l'oreille interne, des traumatismes de la rétine, des glaucomes, des cancers du système nerveux.

### 3-Autres

Les procédés objets de la présente invention peuvent être utilisés pour cribler des molécules pour le traitement ou la prévention des psychoses y compris la schizophrénie, des troubles anxieux, de la dépression, des attaques de panique, des neuropathies périphériques, de la migraine, des tremblements, du désordre compulso-obsessionnel, des désordres thymiques, des dyskinésies tardives, des désordres bipolaires, des désordres du mouvement induit par les médicaments, des dystonies, des chocs endotoxémiques, des chocs hémorragiques, de l'hypotension, de l'insomnie, des maladies immunologiques, des vomissements, des troubles de l'appétit (boulimie, anorexie), de l'obésité, des troubles de la mémoire, dans le sevrage aux traitements chroniques et abus d'alcool ou de médicaments (opioïdes, barbituriques, cannabis, cocaïne, amphétamine, phencyclidine, hallucinogènes, benzodiazépines par exemple), comme analgésiques ou potentialisateurs de l'activité analgésique des médicaments narcotiques et non narcotiques.

### B- Obésité/maladies cardio-vasculaires

La présente invention permet la recherche de composés antagonisant l'activité de GRF1 et jouant un rôle protecteur contre la prise de poids, principalement chez les sujets adultes.

GRF1 interviendrait dans la régulation en amont de la synthèse de la leptine et ceci de façon directe ou indirecte. La leptine est une hormone connue pour inhiber la libération de Neuropeptide-Y (NPY), molécule stimulant l'appétit et produite par les neurones du noyau arqué de l'hypothalamus (20, 21). Elle contrôle également la synthèse d'un peptide anoréxigène : CART (Cocaïne- and Amphetamine- Regulated Transcript) dans le noyau arqué (22). Les activités antagonistes de ces deux neuropeptides ont pour conséquence d'équilibrer les effets du signal leptine sur la prise d'aliments. La leptine stimule également le circuit anoréxigène alpha-MSH/Mélanocortine 4-Recepteur (23-25).

GRF1 est susceptible de participer aux voies de signalisation qu'utilisent ces neuromédiateurs (qui se lient à des récepteurs à sept domaines transmembranaires). Son absence dans nos souris mutantes pourrait se traduire par un signal de type "satiété et/ou augmentation de la dépense énergétique" avec pour conséquence une réduction de la masse adipeuse et une leptinémie basse.

### C- Angiogénèse

L'endothélium vasculaire se présente comme une nouvelle cible de la leptine. Des résultats récents montrent que la leptine stimule la prolifération des cellules endothéliales et l'angiogénèse *in vivo* (26, 27).

L'angiogénèse joue un rôle important dans l'embryogenèse, la cicatrisation, le cycle menstruel mais aussi dans des situations pathologiques comme la vascularisation des tumeurs, l'arthrite rhumatoïde, le psoriasis, le sarcome de Kaposi, les rétinopathies diabétiques et l'athérosclérose.

Les présents inventeurs ont montré que les souris adultes knock-out pour le gène *grf1* ont un taux de leptine bien plus bas que celui des animaux contrôles. Il est possible que les animaux mutants présentent une certaine forme de protection contre l'apparition des processus angiogéniques pathologiques et par voie de conséquence contre la croissance et la dissémination des tumeurs et contre toutes les pathologies précitées.

Des antagonistes de GRF1 qui conduiraient à une diminution de la leptinémie pourraient avoir un rôle protecteur contre ces maladies. Un rôle contraceptif peut aussi être envisagé.

### D- Autres cibles biologiques

La leptine est impliquée dans le déclenchement de la puberté et le contrôle de la reproduction (28-32).

La leptine joue également un rôle dans la régulation de la réponse immunitaire régulée par les lymphocytes T (33).

Des modulateurs de l'activité de GRF1 pourraient avoir un effet sur la fonction immunitaire et les mécanismes de la reproduction. Cette dernière hypothèse semble d'ailleurs se vérifier, puisque les observations des présents inventeurs indiquent une puberté retardée et une ménopause précoce chez les souris *grf1* KO. Ce phénomène peut être dû en partie à la diminution du taux de leptine ou à la dérégulation de la synthèse des hormones sexuelles contrôlées par l'axe hypothalamo-hypophysaire.

Les propriétés inhibitrices de la leptine vis à vis de la synthèse osseuse ont été récemment mises en évidence. Elle agit en inhibant l'activité des ostéoblastes, une population de cellules responsables de la formation de l'os (34). Modifier la leptinémie en agissant sur GRF1 pourrait permettre de traiter les maladies liées à une diminution de la densité osseuse comme par exemple l'ostéoporose ou à l'inverse celles liées à une calcification importante comme par exemple l'ostéopétrose.

Des travaux réalisés sur des souris knock-out pour le gène *NPY* ont montré que ces animaux manifestaient un goût prononcé pour l'alcool et étaient plus résistants à ses effets sédatif et hypnotique que les souris sauvages (35). La leptine joue un rôle négatif sur la libération de NPY (20, 21). Des modulateurs de l'activité de GRF1 pourraient être utilisés dans le traitement du comportement alcoolique et dans le traitement des troubles du sommeil.

D'autres objets, caractéristiques et avantages complémentaires vont être décrits ci-dessous en référence aux exemples de réalisation qui suivent, donnés à titre purement illustratif et non limitatif, et en référence aux dessins annexés dans lesquels :
- la FigurelA est une représentation schématique de la protéine GRF1,
- la Figure 1B est un schéma regroupant les principales voies de signalisation qui impliquent la fonction de la protéine GRF1,
- les Figures 2A et 2B sont des histogrammes représentant des examens neurologiques,
- les Figures 3A à 3B sont des histogrammes illustrant l'étendue des lésions cérébrales respectivement globales et du cortex,
- la figure 3C illustre les lésions des différentes zone de la région corticale,
- la figure 3D illustre le volume des lésions de la région corticale compris entre 3, 22 et 1,76.
- les Figures 4A et 4B illustrent respectivement le poids et la leptinémie de souris mutantes *grf1* KO (notées -/-) et sauvages (notées +/+), et
- les Figures 5A et 5B illustrent respectivement le poids et la leptinémie de souris mutantes KO *grf1* et sauvages après un jeûne de 48 h.

### EXEMPLES

### Exemple 1 : Utilisation de souris porteuses d'une mutation inactivante du gène grf1 (souris dites " knock-out grf1 " ou " KO grf1 ") comme modèle d'étude de la protection contre la mort neuronale induite par ischémie cérébrale.

Les souris KO *grf1* offrent la possibilité de rechercher l'impact des voies dépendantes de la protéine GRF1 sur l'ischémie cérébrale. Des souris KO et de type sauvage sont soumises à une ischémie cérébrale focale permanente. Les paramètres mesurés afin de déterminer l'incidence de GRF1 sur le dommage ischémique résultent (i) d'un examen de la fonction neurologique et (ii) d'une quantification histologique des lésions cérébrales.

### 1.1. : Modèle d'ischémie cérébrale focale permanente

On utilise des souris mâles de 22-37 g issues des fonds génétiques C57B1/6 et 129SV anesthésiées à l'halothane à 1,4 % dans un mélange oxyde nitreux-oxygène (70 : 30). Une incision est réalisée entre l'oeil et l'oreille gauche. Le muscle temporal est récliné. On réalise une craniotomie au niveau de l'os temporal, qui permet d'accéder à l'artère cérébrale moyenne. Celle ci est cautérisée par électrocoagulation. On provoque alors une ischémie cérébrale focale permanente par occlusion de l'artère cérébrale moyenne gauche (MCA.O, (36)). En cours de chirurgie, tant la température du muscle temporal que la température corporelle sont maintenues en normothermie. L'incision est ensuite suturée, et les animaux sont remis dans leurs cages dans une pièce chauffée à 24-26 °C.

Les souris sont divisées en groupes comme suit :

Souris déficientes pour l'expression de GRF1 (souris KO) : C57B1/6 mutants (n = 10), 129SV mutants (n = 21).

Les souris de type sauvage utilisées comme témoins sont C57B1/6 contrôles (n = 10) et 129 SV contrôles (n = 20).

### 1.2 : examen neurologique

Pour l'examen neurologique on procède comme suit :

Avant ischémie, puis 1 h et 24 h après ischémie, on réalise un examen neurologique, initialement décrit chez le rat (37) et légèrement modifié par les présents inventeurs. Lors de l'examen, différents items sont notés : 0 (normal), 1 (flexion de la patte antérieure), 2 (mouvement circulaire), 3 (flexion de la patte antérieure et torsion du thorax), 4 (perte du réflexe de redressement).

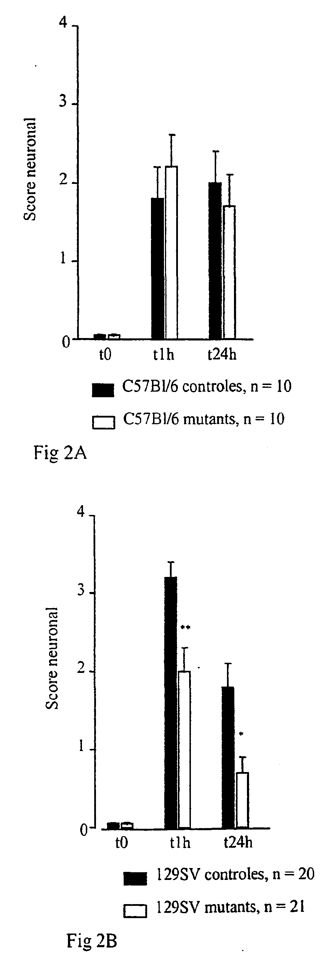

Comme on peut lè constater sur les Figures 2A et 2B, toutes les souris révèlent un déficit neurologique significatif, par comparaison à leur propre score neuronal pré-ischémie, 1 h et 24 h post-MCA.O. Chez les souris C57B1/6, aucune différence n'est observée entre les souris de type sauvage et les souris KO dans l'ensemble des points examinés au cours du temps. A l'opposé, comparées aux souris de type sauvage, les souris 129SV-KO présentent un déficit nettement moins prononcé 1 h (p<0,01) et 24 h (p<0,05) post-ischémie.

### 1.3 : lésion cérébrale.

On procède comme suit : Après notation de leur " neuroscore ", les souris sont sacrifiées et les cerveaux sont rapidement prélevés et congelés dans l'isopentane liquide à - 30 °C. Des sections coronales de 40 µm d'épaisseur sont coupées à différents niveaux stéréotaxiques avec un cryostat, et sont ensuite colorées au cresyl violet à 0,5 %. Des zones de lésion sont mesurées avec un analyseur d'image (Leica Q500) à différents niveaux coronaux. Le volume des lésions cérébrales est ensuite calculé par intégration des surfaces.

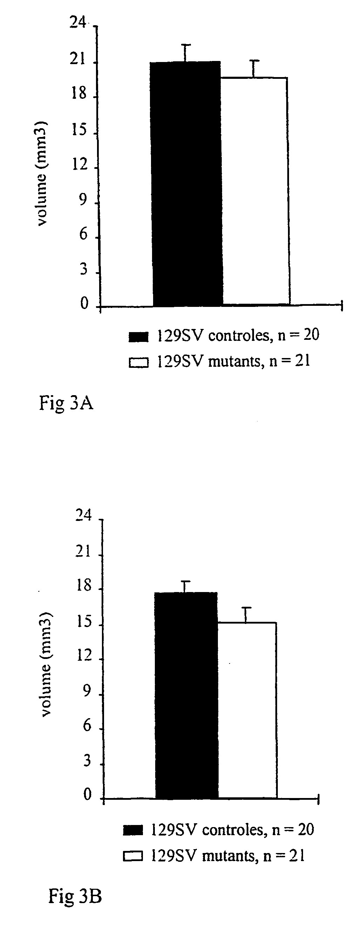

Chez les souris C57B1/6, on n'observe pas de différence significative entre les souris de type sauvage et les souris KO (résultats non illustrés).
Comme on peut le constater aux Figures 3A à 3B, chez les souris 129SV, on n'observe pas de réduction significative globale ou corticale de la lésion cérébrale.

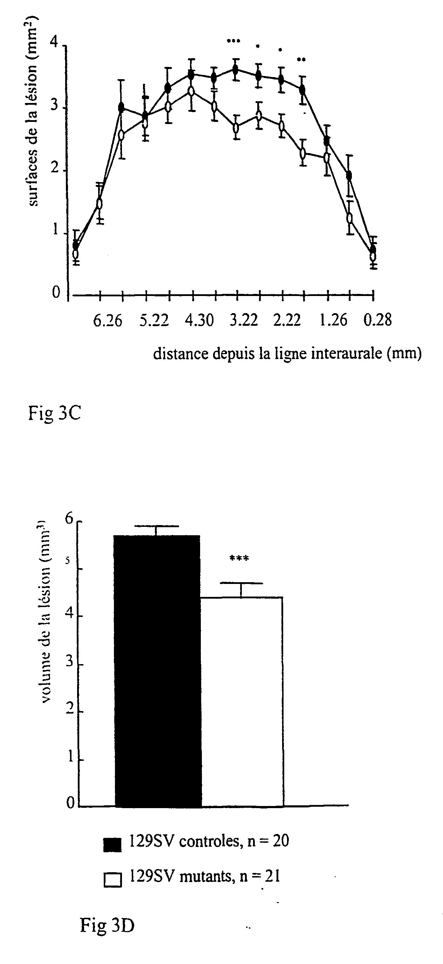

Toutefois, on note des réductions significatives des zones de lésion à certains niveaux stéréotaxiques (fig 3C), ce résultat correspondant à une réduction de 23 % (p<0,05) de la lésion dans cette région spécifique (fig 3D).

Les résultats décrits à l'Exemple 1 ci-dessus montrent que l'absence du gène *grf1* a pour conséquence une meilleure récupération fonctionnelle chez les souris ischémiées KO-129SV.

La réduction des lésions dans des niveaux coronaux spécifiques pourrait correspondre aux structures impliquées dans les fonctions motrices et sensori-motrices examinées (notamment le néocortex et le cervelet), dans lesquelles le gène *grf1* pourrait être fortement exprimé (12). A l'opposé on ne remarque pas d'effet chez les souris de fond génétique C57B1/6.

L'inactivation du gène *grf1* pourrait interrompre une des voies de signalisation conduisant à l'apoptose. Autrement dit, les résultats rapportés ci-dessus suggèrent que la protéine GRF1 participe à la signalisation conduisant à l'apoptose neuronale en intervenant dans la voie d'activation des kinases de stress par activation d'une ou plusieurs des petites protéines G de la famille des protéines Rho, Rac et Cdc42. Il a d'ailleurs été décrit l'activité de GRF1 sur la protéine Rac (2).

En résumé, la protéine GRF1 pourrait activer en partie une voie apoptotique responsable de la vulnérabilité à l'ischémie. Ces résultats suggèrent en outre que la protéine GRF1 elle-même pourrait représenter une cible en particulier pour des désordres neurodégénératifs aigus.

### Exemple 2 : Utilisation de souris porteuses d'une mutation inactivante du gène grf1 (souris dites " knock-out grf1 " ou " grf1 KO") comme modèle d'étude de la protection contre l'obésité.

### 2.1 : Obésité

Des souris sauvages âgées de 10-12 mois, nourries depuis le sevrage avec une alimentation destinée à l'élevage, riche en protéines (22 %) et en matières grasses (7 %) (réf: D03, Code : R0310. Fournisseur : UAR, France), présentent une masse adipeuse importante entre la peau et la tunique musculaire abdominale, ainsi que dans la cavité abdominale, principalement autour des cornes utérines chez les femelles et dans le péritoine. La présence de graisse autour du coeur est également trouvée chez les sujets les plus gros que l'on peut qualifier d'obèses et dont le poids dépasse 35 grammes chez les femelles et 40 grammes chez les mâles.

Dans les mêmes conditions d'élevage et de nutrition, les souris porteuses de la mutation inactivante pour le gène *grf1* âgées d'un an et plus ne présentent aucune des caractéristiques décrivant l'obésité. La majorité d'entre elles n'ont pas de tissu adipeux entre la peau et la paroi abdominale. Chez les femelles, le dépôt de graisse autour des cornes utérines est très faible et comparable en quantité à celui d'animaux sauvages de 6-8 semaines. Les souris *grf1* KO adultes se caractérisent encore par l'absence de graisse dans le péritoine et autour du coeur.

On observe que l'inactivation du gène *grf1* chez la souris adulte, se traduit par une protection contre la prise de poids et l'accumulation de tissu adipeux.

### 2.2 : leptinémie

Afin de corréler les observations faites chez les souris *grf1* KO et sauvages avec des caractéristiques connues du phénotype obèse, nous avons dosé la leptine plasmatique des souris sauvages et mutantes.

La leptine est une cytokine qui a pour effet de diminuer la prise d'aliments et d'augmenter la dépense énergétique. Il existe une relation de proportionnalité entre d'une part, le nombre d'adipocytes - qui sécrètent la leptine - et la taille des vésicules lipidiques qu'ils contiennent et d'autre part, la concentration plasmatique de cette hormone (20).

Les présents inventeurs ont déterminé le poids et la leptinémie de souris mâles et femelles sauvages ou mutantes (KO) pour le gène *grf1*, âgées d'un an. Le groupe d'animaux étudié est constitué de plusieurs fratries. Chaque fratrie est composée d'animaux sauvages et mutants.

Les échantillons sanguins ont été prélevés lors de l'euthanasie des animaux par décapitation.

Le dosage de la leptine plasmatique a été réalisé en utilisant un radioimmunoessai (RIA) fabriqué par la société Linco, sous la forme d'un kit (ref: ML-82K) qui est commercialisé en France par la société Clinisciences.

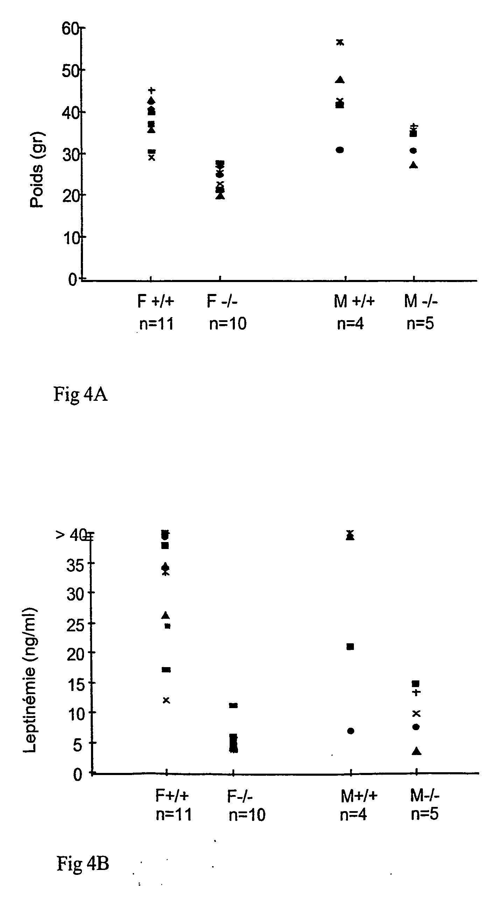

Comme on peut l'observer aux Figures 4A et 4B chez les souris mutantes (notées -/-), la teneur plasmatique en leptine est très faible comparée à celle des souris sauvages témoins du même âge (notées +/+). Si on se réfère à la littérature, la leptinémie des souris *grf1* knock-out âgées de plus de 12 mois et comparable à celle des souris très jeunes de 6-8 semaines (5 à 10 ng/ml). L'examen comparatif de la silhouette de ces animaux abonde dans ce sens.

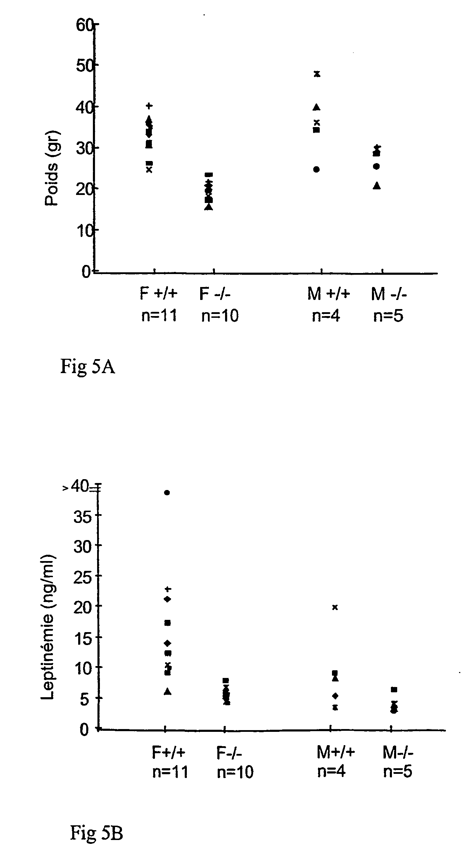

Si les animaux sont mis à jeun pendant 48h (Figures 5A et 5B), il s'en suit une diminution du poids corporel accompagnée chez les animaux contrôles, d'une diminution importante de la concentration plasmatique en leptine, tandis que chez les animaux KO la diminution de la leptinémie est très faible. Ce dernier résultat suggère que chez les animaux KO, la teneur en leptine est déjà à un seuil minimum qui ne peut être modulée que faiblement en réponse à un jeûne prolongé.

Les premières mesures de la consommation d'aliments chez les souris mutantes indiquent que la prise d'aliments est réduite par rapport aux contrôles. Cependant si on ramène la quantité de nourriture absorbée au poids des animaux il n'y a pas de différence significative entre les KO et les sauvages.

Il est à noter que si le phénotype des souris mutantes a pour origine une augmentation de la dépense énergétique, cette dernière ne semble pas due à une hyperactivité des souris *grf1* KO.

### Exemple 3 : Utilisation de la protéine GRF1 pour la réalisation de cribles de composés par mesure de la phosphorylation en sérines/thréonines ou en tyrosines de la protéine GRF1.

Cette expérience est réalisée sur des cellules en culture.

L'induction de la phosphorylation de GRF1 est induite dans ces cellules en culture de différentes façons.

Trois protocoles sont utilisés :

### 1) Marquage radioactif et immunoprécipitation de la protéine GRF1

Ces différents traitements sont faits en présence et en absence des molécules à cribler, dans du milieu de culture contenant de l'orthophosphate marqué au P³² ou au P³³( 200µCi à 1 mCi / boîte de Pétri de 10 mm ). La lyse des cellules se fait à froid dans un tampon HNTG (Hepes 20 mM pH 7,5, NaCl 150 mM, Triton 0,1 %, Glycérol 10%, en présence d'inhibiteurs de protéases et de phosphatases : 1mM Na3VO4, Aprotinin 2,2 µg/ml,1 µg/ml Leupeptin, 1 µg/ml Antipain, 10 µg/ml Benzamidine, 1 µg/ml Inhibiteur de trypsine du soja, 1 µg/ml Chymostatin, 1 µg/ml Pepstatin-A) ou RIPA (50 mM Tris, pH 8,0, NaCl 150 mM, 1% NP-40, 0,5% NaDeoxycholate, 0,1% SDS, 1 mM Na3VO4, 100µM phenylméthylsulphonyl fluorure (PMSF), 25µg/ml Aprotinin, 25µg/ml Leupeptin). La protéine GRF1 est ensuite immunoprécipitée à l'aide d'anticorps anti-GRF1 ou anti-tag (10), (épitope reconnu par un anticorps monoclonal et permettant la détection ou l'immunoprécipitation de la protéine étiquetée (tagged en anglais) comme FLAG, myc, ou hemagglutinin (HA)), et l'immunoprécipitat est séparé sur un gel SDS-polyacrylamide 4-20 %. La quantification de la radioactivité de la bande correspondant à la protéine GRF1 (140 kDa) est réalisée à l'aide d'un détecteur de rayonnement de type PhosphoreImager. L'efficacité d'une molécule se calcule par le pourcentage d'inhibition de la phosphorylation de la protéine GRF1 en considérant la valeur trouvée pour les cellules non stimulées comme la valeur 0 % et la valeur trouvée pour les cellules stimulées en l'absence de lamolécule criblée comme la valeur 100 %.

### 2) Marquage radioactif et rétention de la protéine GPF1 dans des plaques CYTOSTAR

Une première alternative à cette méthode consiste à incuber le lysat cellulaire obtenu précédemment dans des puits de plaques CYTOSTAR (commercialisées par la société Amersham) préalablement recouverts d'un anticorps anti-GRF1 ou anti-tag afin d'y retenir la protéine GRF1. Après rinçages, la quantification du signal radioactif est obtenue à l'aide d'un compteur à scintillation.

### 3) Quantification par E.L.I.S.A

Une deuxième alternative à cette méthode consiste à traiter les cellules comme précédemment mais en l'absence d'orthophosphate marqué. Les lysats cellulaires sont alors utilisés selon la méthode de dosage E.L.I.S.A. Pour ce dosage, les puits des plaques sont recouverts d'un anticorps anti-GRF1 ou anti-tag et la phosphorylation de GRF1 est révélée et quantifiée par un deuxième anticorps marqué (radioélément, fluorescence, enzyme, etc) ou non qui peut être soit un anti-phosphotyrosine, soit un anti-phosphosérine/phosphothréonine. Dans le cas où le second anticorps n'est pas marqué, la révélation et la quantification se font à l'aide d'un troisième anticorps anti-espèce dirigé contre le second et marqué. Après rinçage, la quantification du signal se fait à l'aide d'un compteur de radioactivité, d'un spectrophotomètre de fluorescence, ou par la mesure d'une activité enzymatique, qui donne lieu à une lecture de densité optique par spectrophotomètre (lecteurs de plaques).

Les cellules en culture, peuvent être:
■ des cultures primaires de cellules neuronales, maintenues 10 jours en culture afin de permettre l'expression de la protéine GRF1 endogène
■ des lignées cellulaires en culture de toute origine tissulaire transfectées avec un vecteur permettant l'expression de la protéine GRF1 (murine ou humaine), entière et éventuellement étiquetée (tagged en anglais). Tout vecteur plasmidique ou viral permettant l'expression de cDNAs hétérologues dans des cellules mammifères peut être utilisé.

La phosphorylation de la protéine GRF1 dans les cellules en culture est obtenue dans les cellules neuronales, par addition de 100µM de Carbachol (5 à 30 minutes) dans le milieu de culture des neurones.

Pour les cellules non neuronales transfectées par le cDNA codant pour GRF1, la phosphorylation de la protéine d'intérêt est étudiée dans les conditions suivantes :
■ Soit les cellules transfectées sont carencées en sérum sur la nuit, puis réincubées en présence de 10 % sérum de veau foetal,
■ Soit les cellules sont cotransfectées par les cDNAs codant pour les sous unités béta-gamma des protéines G trimériques β1γ2 ou β1γ5. Les vecteurs d'expression sont des plasmides permettant l'expression dans des cellules eucaryotes sous le contrôle de promoteurs du type CMV ou SV40 (exemples pSV2 ou bien pCDNA3 (INVITROGEN)). Les cDNAs codant pour les protéines d'intérêt y sont insérés après amplification des fragments par PCR, vérification de la séquence et sous-clonage des fragments dans les sites multiples de clonage présents dans les vecteurs. Les cellules sont déprivées en sérum sur la nuit, puis réincubées en présence de sérum.

### Exemple 4 : Utilisation de la protéine GRF1 pour la réalisation de cribles de composés par mesure de l'activité d'échange sur la protéine Ras ou les protéines Rac ou Cdc42 en présence de GRF1.

Ces activités peuvent se mesurer dans des systèmes acellulaires (utilisation de protéines recombinantes) ou dans des systèmes cellulaires.

### 1) Mesure dans des systèmes acellulaires

Les protéines Ras, Rac ou Cdc42 recombinantes à la concentration finale 0,5 µM, sont incubées en présence de ³H-GDP (0,5 µM final, NEN 111 µmoles, 9 Ci/mmoles) dans le tampon d'échange (50 mM tris-HCl, pH 7,5, 1 mM MgCl₂, 10 mM dithiothreitol,1mM EDTA, 1mg/ml sérum albumine bovine) pendant 30 minutes à 30°C, puis conservées dans la glace. On ajoute ensuite du MgCl₂ pour avoir une concentration finale de 10mM afin de bloquer la réaction d'échange puis on prélève 50 µl de chaque incubation (Ras/Rac/Cdc42 lié au GDP tritié) qui sont distribués dans l'un des 96 puits d'une micro-plaque.

La réaction d'échange est démarrée avec l'addition dans les puits de 10 µl de GTP froid 10 mM (0,1 mM final), de la protéine GRF1 recombinante (tout ou parties) et des molécules à tester.

Après une incubation de 60 minutes à 30°C, un aliquot de 40 µl est prélevé et filtré sur une membrane de nitrocellulose de 0,45 µm (plaque 96 puits à fonds recouverts d'une membrane de filtration pouvant retenir les protéines, Sartorius SM 11306). Le comptage de la radioactivité (GDP tritié lié à la petite protéine G) est effectuée dans un compteur à scintillation, en présence de liquide scintillant. Dans ce cas, c'est la radioactivité retenue sur la membrane de filtration de nylon qui est comptée.

Une alternative à la réaction de filtration consiste à passer, après incubation, le contenu de chaque puits sur une colonne PD10 préalablement équilibrée avec le tampon d'élution (50mM Tris-HCl pH 7,5 , 5 mM MgCl₂, 1 mM PMSF, 5 mM DTT et 1 mM EDTA). Dans ce cas, c'est la radioactivité de l'éluat de la colonne qui est mesurée (4 ml de tampon d'élution passé sur la colonne + 10 ml de liquide scintillant ReadyGel Beckman).

Dans les deux cas, l'activité d'échange est mesurée par la différence de quantité de radioactivité en présence de protéine GRF1 comparée à celle obtenue dans un essai sans protéine GRF1. L'efficacité d'une molécule se calcule par l'inhibition de cette activité d'échange.

Les protéines Ras, Rac ou Cdc42 recombinantes peuvent être des protéines Ras, Cdc42 ou Rac sauvages de différentes origines (humaine, murines,..) exprimées :
soit sous forme de protéines de fusion (GST, système pGEX-1λT ou -2T, Produit Pharmacia) dans E. coli (selon le protocole décrit par le fabricant et après transformation des plasmides dans les bactéries BL21. L'expression des protéines est induite pendant 4 heures avec 0,5 mM d'IPTG (isopropyl-1-thio-beta-D-galactoside) à 30°C) ou dans des cellules de mammifères (pBCGST, (38)) dans les cellules CHO transfectés par les plasmides. L'expression est obtenue à 37°C dans une étuve à 5% CO2 sur la nuit. Les protéines sont purifiées sur colonne d'affinité de glutathion selon les protocoles fournis par Pharmacia (Biotech (GST-Gene Fusion system). Ces protéines de fusion peuvent être on non clivées par digestion à la thrombine afin de supprimer leur domaine GST.
soit sous une forme étiquetée (tagged en anglais) (pBlueBacHis2, Produit Invitrogen) dans baculovirus, exprimée dans des cellules d'insectes (Sf9, Sf21, High Five, ...) après infection des cellules par un baculovirus recombinant obtenu selon les procédés recommandés par le vendeur. L'expression se fait à 27 °C sur deux à quatre jours et les protéines sont ensuite purifiées sur colonne de nickel.

Les protéines GRF1 recombinantes peuvent être la totalité ou des fractions de protéines GRF1 de différentes origines (humaine, murine,..) contenant les domaines d'échange soit CDC25, soit DH, soit les deux, exprimées dans baculovirus ou dans *E. coli*, et purifiées sur colonne d'affinité.

Une variante de la technique précédente consiste à utiliser la capacité soit de Ras-GTP à se lier à Raf-1 par le domaine « Ras-Binding domain » de Raf (RBD), soit de Racl-GTP ou Cdc42-GTP à se lier à PAK1 par le domaine CRIB de cette dernière (39). Le système SPA (« Scintillation Proximity Assay »), décrit dans US 4 568 649, EP 154 734et JP 84/52452, permet de mettre en évidence et de quantifier la liaison entre deux protéines dans un crible à haut flux.

Les protéines Ras, CDC42 ou Rac recombinantes - dans cette expérience, les petites G seront clivées de leur domaine GST ou bien exprimées avec une autre étiquette (tag en anglais) par exemple, queue His,...- à la concentration finale 0,5 µM, sont incubées en présence de GDP froid dans le tampon d'échange (50 mM Tris-HCl, pH 7,5, 1 mM MgCl₂, 10 mM dithiothreitol,1mM EDTA, 1mg/ml sérum albumine bovine) pendant 30 minutes à 30°C, puis conservées dans la glace. Les étapes suivantes consistent à ajouter du MgCl₂ jusqu'à une concentration finale de 10 mM pour bloquer la réaction d'échange, puis à prélever 50 µl de chaque incubation et à les distribuer dans l'un des 96 puits d'une micro-plaque.

La réaction d'échange est démarrée avec l'addition dans les puits de 10 µl de GTP tritié (NEN 111 µmoles, 9 Ci/mmoles), de la protéine GRF1 recombinante et des molécules à tester.

Après une incubation de 60 minutes à 30°C, ajouter 80 µl de protéines GST-Raf (RBD domain) ou GST-PAK (CRIB domain) à la concentration de 0,0013 mg/ml dans un tampon 50mM Tris-HCl, pH 7,5, 2 mM dithiothreitol et 40 µl d'un mélange d'immunoglobuline anti-GST (0,12 mg/ml) et de protéine A PVT SPA couplée à des micro-sphères (Amersham ; 12,6 mg/ml) dans un tampon 50mM Tris-HCl, pH 7,5, 2 mM dithiothreitol, 2 mM MgCl₂. Les plaques sont ensuite scellées, mises sous agitation 1 h à 22°C, puis centrifugées à 760 g pendant 2 minutes et comptées dans un compteur à scintillation.

L'efficacité de la molécule se calcule par le pourcentage d'inhibition de l'activité d'échange et est égale à : 100 x (1 - (valeur en présence de GRF1 et de la molécule - témoin en absence de GRF1 et de la molécule) / (valeur en présence de GRF1 et en absence de la molécule - témoin en absence de GRF1 et de la molécule)).

### 2) Mesure dans des systèmes cellulaires (levure)

Différentes possibilités sont envisageables. Elles utilisent soit les systèmes de complémentation de la levure (*Saccharomyces cerevisiae*), soit des systèmes de compétition produisant un désordre physiologique, soit le système double-hybride. Toutes les techniques et méthodes qui utilisent la levure sont largement décrites dans la littérature (40-45).

L'utilisation de l'un ou l'autre de ces systèmes est envisageable du fait de l'existence chez la levure de couples protéine G de petit poids moléculaire/ facteur d'échange, homologues à ceux décrits chez les cellules de mammifères. Pour ce qui est des protéines G, les protéines RAS de levure sont homologues aux protéines Ras de mammifères (ces dernières complémentent dans la levure les mutations inactivantes des protéines RAS) et la protéine CDC42 *S.c.* est homologue aux protéines Rac mammifères. En ce qui concerne les facteurs d'échange, CDC25 et SDC25 sont homologues à GRF1 et leurs parties catalytiques sont actives sur les protéines Ras de mammifères (46, 47). GRF1, par son domaine homologue à CDC25, est capable de complémenter toute mutation inactivante de CDC25, ou bien d'un double mutant CDC25/SDC25 (les mutants ou KO SDC25 n'ayant pas de phénotype). On connaît aussi pour CDC42 *S.c.* un facteur d'échange appelé CDC24 qui peut être complémenté par le domaine DH de Vav (48). On peut extrapoler une activité d'échange de tout ou partie (incluant le domaine DH) de GRF1 sur CDC42 dans une levure mutante pour CDC24, ces mutations inactivantes ayant un phénotype létal. Cette activité de GRF1 (tout ou partie) mimerait totalement ou partiellement (selon son affinité) l'effet de CDC24.

### 2.1 Complémentation dans la levure

Dans ce système, GRF1 (tout ou parties) est capable de mimer la fonction des facteurs d'échange de levure (CDC25, CDC24) lorsque ceux-ci sont inactivés par une mutation conduisant à un phénotype du type arrêt de la croissance. Cette complémentation a pour effet de permettre la survie de la levure en restaurant la croissance. Une molécule inhibant la fonction de GRF 1 (protéine entière, domaine CDC25, domaine DH) sur les protéines RAS ou CDC42, dans un contexte CDC25 ou CDC24 inactivés (mutations thermosensibles) conduit à la perte du phénomène de complémentation et à la mort de la cellule à température permissive. La spécificité du mode d'action de la molécule sur le système RAS/ domaine CDC25 ou CDC42/ domaine DH sera vérifié par la mesure de l'action de la molécule sur une levure sauvage (information sur l'activité antifongique).

Le criblage se fera pour les petites molécules dans une levure perméabilisée par des techniques connues de l'homme de l'art consistant à élever la concentration intracellulaire des molécules en jouant sur la perméabilité membranaire et sur l'expression de gènes impliqués dans les processus de détoxification (par exemple en utilisant des mutants du gène Erg6 et/ou des gènes de la famille PDR) Ces techniques, dites de perméabilisation, sont décrites dans les demandes de brevet suivantes : FR 9411509 et WO 96/1082.

### Souches de levures utilisées

Une souche du genre *S.cerevisiae,* CDC25 TS ou CDC24 TS (mutations thermosensibles) est utilisée. Elle ne peut croître que dans des conditions de température permissive. Elle est cultivée sur le milieu de culture suivant :
Milieu YNB minimum :-Yeast Nitrogen Base (sans acides aminés) (6,7g/l) (Difco)
   - Glucose (20g/l) (Merck)

Ce milieu peut être rendu solide par addition de 20g/l d'agar (Difco).

Pour permettre la croissance des levures auxotrophes sur ce milieu, il est nécessaire d'y ajouter les acides aminés ou les bases azotées, desquelles elles sont dépendantes à 50mg/ml. 100 µg/ml d'ampicilline sont ajoutés au milieu afm d'éviter les contaminations bactériennes.

### Construction et amplification des plasmides

La souche TG1 d'Escherichia coli, de génotype supE, hsdΔ5, thi, Δ(lac-proAB), F'[tra D36 pro A⁺B⁺ lacI^{q} lacZΔM15], est employée pour la construction de plasmides, et pour l'amplification et l'isolement de plasmides. Elle est cultivée sur le milieu suivant:
Milieu LB : - NaCL(5g/l) (Sigma)
   - Bactotryptone (10g/l)(Difco)
   - Extrait de Levure (5g/l)(Difco)

Ce milieu peut être rendu solide par addition de 20g/l d'agar (Difco).

De l'ampicilline, à 100 µg/ml, est utilisée pour sélectionner les bactéries ayant reçu les plasmides portant comme marqueur le gène de résistance à cet antibiotique.

Les préparations en petites quantités et en grandes quantités d'ADN plasmidique sont effectuées selon les protocoles recommandés par le fabricant Quiagen des kits de purification d'ADN :
- Quiaprep Spin Miniprep kit, ref : 27106
- Quiaprep Plasmid Maxiprep kit, ref : 12163.

### Transformation de la levure par un plasmide

Les levures sont rendues compétentes par un traitement au LiAC/PEG d'après la méthode décrite par Gietz *et al*, (49) et transformées par 1µg de plasmide permettant l'expression constitutive ou inductible de tout ou partie de GRF1 dans la levure. Après les étapes de transformation, les levures sont remises en culture dans des conditions non permissives. Les clones sélectionnés seront ceux qui auront été complémentés par GRF1.

### Criblage des molécules

Un clone sélectionné complémenté par GRF1 est étalé sur des boîtes contenant le milieu de sélection sur lequel des gouttes de composés chimiques sont appliquées. Les boîtes sont placées en conditions permissives. Les produits donnant un halo d'inhibition de croissance de la levure, sont retenus et testés sur une levure sauvage de contrôle pour éliminer les molécules à activité antifongique qui donnent le même type d'inhibition de croissance.

### 2.2 Compétition avec les GEFs de levure (deux variantes)

### a- Activations constitutives / effet dominant positif :

Dans ce système, l'expression de GRF1 tout ou partie, dans la levure s'accompagne d'une perturbation de la croissance du fait de la stimulation constitutive de l'échange sur les protéines RAS ou CDC42. Une molécule active sur le système conduira à une normalisation plus ou moins parfaite du désordre physiologique (phénotype) engendré par l'expression de GRF 1.

### Souches utilisées

Une souche de levure sauvage du genre *S.cerevisiae* est utilisée. Elle est cultivée sur le milieu YNB minimum comme décrit en 2.1. La construction et l'amplification des plasmides sont effectués comme décrit en 2.1.

Dans l'exemple décrit ci-après, l'expression de GRF1 conduit à la suractivation de RAS de levure. De telles levures ne seront alors plus capable de fabriquer des réserves de glycogènes lorsqu'elles atteindront la phase de croissance pré-stationnaire. Elles seront identifiées par l'absence de coloration brune après exposition aux vapeurs d'iode.

### Criblage des molécules

Le criblage se fera dans une levure perméabilisée selon les techniques décrites précédemment.

Des gouttes de composés chimiques sont appliquées directement à la surface de boîtes de culture préalablement ensemencées avec une levure exprimant GRF1, tout ou partie. Les boîtes sont mises à cultiver puis exposées aux vapeurs d'iode dans une enceinte fermée contenant des cristaux d'iode. Les produits permettant la croissance de la levure et qui restaurent l'apparition de la coloration brune, seront retenu comme positifs.

### b- Inhibitions constitutives / effet dominant négatif :

On peut également envisager que l'expression de GRF1, tout ou partie, se comporte chez la levure comme un dominant négatif, inhibe l'activité des protéines de levure CDC25 ou CDC24 et provoque un désordre physiologique du type arrêt de la croissance, qui sera levé totalement ou partiellement par des molécules actives sur le système.

### Souches utilisées

Une souche sauvage du genre *S.cerevisiae* est utilisée. Elle est cultivée sur le milieu YNB minimum comme décrit en 2.1. La construction et l'amplification des plasmides sont effectués comme décrit en 2.1.

### Transformation de la levure par un plasmide

La levure sauvage est rendue compétente par un traitement au LiAC/PEG d'après la méthode décrite par Gietz *et al*, (49) et transformée par 1µg de plasmide permettant l'expression inductible de tout ou partie de GRF1 dans la levure (exemple : l'expression du gène *grf1* est placée sous le contrôle du promoteur du gène Gal 4 qui est inductible en présence de galactose comme seul source de carbone dans le milieu de culture).

Dans l'exemple décrit ci-après et dans les conditions d'inductions, l'expression de GRF1 conduit à la perturbation de la signalisation cellulaire qui utilise les protéines RAS dans la levure et se traduit par un phénotype de type arrêt de la croissance.

### Criblage des molécules

Le criblage se fera dans une levure perméabilisée selon les techniques décrites précédemment.

Des gouttes de composés chimiques sont appliquées directement à la surface de boîtes de culture ensemencées avec une levure transformée par le plasmide d'expression inductible de GRF1, mais préalablement cultivée en absence d'agent inducteur. Les boîtes sont mises à incuber dans les conditions qui permettent l'expression de GRF1. Les produits permettant la croissance de la levure seront retenus comme positifs.

On peut envisager de réaliser ces systèmes de compétition dans des levures mutantes (mutants thermosensibles) pour RAS (RAS mut TS) complémentées avec un Ras de mammifère ou CDC42 mut TS complémentées par Rac de mammifère.

### 2.3 - Systèmes double-hybride et reverse double-hybride / interaction protéine-protéine

### 1^{ère} application :

L'utilisation de ce système permet de réaliser un crible sur des molécules pouvant inhiber l'interaction physique entre Ras ou Rac/CDC42Hs et GRF1 (50, 2).

La technique repose sur la réalisation d'une première protéine de fusion entre Ras, CDC42Hs ou Rac et un domaine d'interaction à l'ADN et d'une seconde protéine de fusion entre GRF1 (tout ou domaines CDC25-like ou DH) et un domaine transactivateur. L'interaction entre les deux protéines hybrides conduit à l'activation d'un gène rapporteur (HIS3, LEU2, URA3, CYH2, CAN1, LacZ, GFP, etc.). Le système est utilisé dans la levure appropriée (ex : mutant ura3 si le gène rapporteur est URA3).

Exemple d'un système reverse double-hybride pour le crible de molécules (51): Le souche de levure CL9 est utilisée comme outil de criblage de molécules interférants dans le système double-hybride. Elle permet la mise en évidence d'interaction protéine-protéine. Elle peut être rendue perméable par introduction de mutations dans la famille des gènes PDR et du gène ERG6 impliqués dans les processus de détoxification.

Elle est cultivée sur le milieu YNB minimum.

La souche TG1 d'Escherichia coli, de génotype supE, hsdΔ5, thi, Δ(lac-proAB), F'[tra D36 pro A⁺B⁺ lacI^{q} lacZΔM15], est employée pour la construction de plasmides, et pour l'amplification et l'isolement de plasmides.

Les plasmides mis en oeuvre sont les suivants :

Le vecteur pGAD10 , fourni par Clontech® permet l'expression chez la levure d'une protéine de fusion entre GRF1, tout ou parties, et le domaine transactivateur de GAL4 (protéine GRF1-TA).

Le vecteur pGBT9, fourni par Clontech® permet l'expression chez la levure d'une protéine de fusion entre Ras, Rac ou Cdc42 et le domaine d'interaction à l'ADN de GAL4 (protéines Ras-BD ou Rac-BD ou Cdc42-BD). Remarque : de façon avantageuse, le domaine carboxy-terminal de farnesylation de Ras ou de géranyl-géranylation des proteines Rac et Cdc42, sont éliminés de la construction. Ce qui permet d'obtenir des protéines qui ne s'accrochent pas aux membranes lipidiques et qui entrent efficacement dans le noyaux des cellules.

### Transformation de la levure par un plasmide

Le levure CL9 est rendue compétente par un traitement au LiAC/PEG d'après la méthode décrite par Gietz *et al,* (49). Elle est ensuite transformée par 1µg de chacun des plasmides permettant l'expression des protéines de fusion qui composent le système double-hybride. L'expression de celles -ci conduit à une sensibilité de la souche au cycloheximide.

Un produit interférant avec l'interaction entre les protéines de fusion du système double-hybride permettra la croissance de la levure sur ce type de milieu. Pour faire un criblage, on étale le levure à la surface d'un milieu sélectif contenant 10µg/ml de cycloheximide. Des gouttes de composés chimiques sont appliquées directement à la surface de la boîte. Les produits sélectionnés sont ceux qui donnent une auréole de croissance autour du dépôt.

Le criblage se fera dans une levure perméabilisée selon les techniques décrites précédemment.

Le criblage se fera dans une levure perméabilisée selon les techniques décrites précédemment.

Dans un autre système l'inhibition de l'intercation protéine-protéine par des molécules actives peut être révélée par la diminution de l'expression d'un gène rapporteur. Celle-ci pouvant, selon le cas, être mise en évidence par un test colorimétrique, fluorimétrique ou enzymatique (exemple : β-galactosidase). L'inhibition de la croissance peut être mesurée par l'utilisation d'un milieu sélectif (ex : milieu au fluorate pour l'utilisation du gène rapporteur URA3 ou milieu à la canavanine pour CAN1).

La mesure de l'activité β-galactosidase se réalise de la façon suivante :

La souche L40 du genre *S.cerevisiae* (*Mata, his3D200, trp1-901, leu2-3,112, ade2, LYS2:: (lexAop)4-HIS3, URA3:: (lexAop)8-LacZ, GAL4, GAL80)* est utilisée comme outil de criblage. Cette souche permet la mise en évidence d'interaction protéine-protéine quand l'un des partenaires protéiques est fusionné à la protéine LexA (70). Elle a été cultivée sur le milieu de culture YNB minimum.

La souche TG1 d'Escherichia coli, de génotype supE, hsdD5, thi, D(lac-proAB), F'[tra D36 pro A⁺B⁺ lacI^{q} lacZDM15], a été employée pour la construction de plasmides, et pour l'amplification et l'isolement de plasmides (comme décrit en 2.1)

Les plasmides mis en oeuvre sont les suivants:

Le vecteur pGAD10, fourni par Clontech® permet l'expression chez la levure de protéines de fusion entre GRF1, tout ou parties et le domaine transactivateur de GAL4.

Le vecteur pLex9 (pBTM116) permet l'expression chez la levure de protéines de fusion entre Ras, Rac ou Cdc42 et le domaine d'interaction à l'ADN de la protéine LexA.

La levure est rendue compétente par un traitement au LiAC/PEG d'après la méthode décrite par Gietz *et al*, (49). Elle est ensuite transformée par 1µg de chacun des plasmides permettant l'expression des protéines de fusion qui composent le système double-hybride. L'expression de celles -ci conduit à l'expression de la β-galactosidase.

Une feuille de nitrocellulose est déposée sur la boîte de Pétri contenant le tapis de levures et les gouttes de composés chimiques à cribler. Cette feuille est ensuite plongée dans de l'azote liquide pendant 30 secondes afin de faire éclater les levures et de libérer ainsi l'activité β-galactosidase. Après décongélation, la feuille de nitrocellulose est déposée, colonies vers le haut, dans une autre boite de Pétri contenant un papier Whatman préalablement imbibé de 1,5ml de solution PBS (Na₂HPO4 60mM, NaH₂PO₄ 40mM, KCl 10mM, MgSO₄ 1mM, pH7) contenant 15 µl de X-Gal (5-bromo-4-chloro-3-indoyl-β-D-galactoside) à 40mg/ml dans de la N,N-diméthylformamide. La boîte est ensuite placée dans une étuve à 37°C. Le test est dit positif lorsque les colonies virent au bleu sur la membrane au bout de 6 heures.

A noter que dans le cas d'interactions faibles ou très fugaces entre protéines, il est possible d'envisager d'introduire des mutations dans celles-ci afin de rendre l'interaction plus forte (52).

### Deuxième application :

Cette application utilise les propriétés d'interaction de PAK1 (domaine CRIB) avec Rac et Cdc42 quand ces petites protéines G sont sous forme liées au GTP et de c-Raf1 (domaine RBD) avec Ras-GTP. On peut alors envisager une variante du système double hybride appelé double-hybride plus un qui consisterait à exprimer dans le noyau d'une même levure soit :
GRF1 (tout ou partie), PAK1 (CRIB domain) fusionnée avec un domaine transactivateur de GAL4 et soit Rac soit Cdc42 Hs fusionnées avec un domaine d'interaction à l'ADN de GAL4 soit
GRF1 (tout ou partie), c-Raf1 (RBD) fusionnée avec un domaine transactivateur de GAL4 et Ras fusionnée avec un domaine d'interaction à l'ADN de GAL4.

Le domaine carboxy-terminal de farnesylation de Ras ou de géranyl-géranylation des proteines Rac et Cdc42, peuvent être éliminés de la construction, ce qui permet d'obtenir des protéines qui ne s'accrochent pas aux membranes lipidiques et qui entrent efficacement dans le noyaux des cellules.

L'activation des petites G par GRF1 induirait leur charge en GTP et donc leur interaction avec leur effecteur (PAK1 ou Raf-1) et permettrait donc l'expression du gène rapporteur comme décrit ci-dessus.

L'inhibition de l'activité d'échange de la protéine GRF1 par des petites molécules se traduirait donc par l'inhibition de l'expression du gène rapporteur. (voir ci-dessus).

Le souche de levure CL9 est utilisée comme outil de criblage de molécules interférants dans le système double-hybride. Elle permet la mise en évidence d'interactions protéine-protéine. Elle peut être rendue perméable par introduction de mutations dans la famille des gènes PDR et du gène ERG6 impliqués dans les processus de détoxification.

Elle a été cultivée sur le milieu YNB minimum.

La souche TG1 d'Escherichia coli, de génotype supE, hsdΔ5, thi, Δ(lac-proAB), F'[tra D36 pro A⁺B⁺ lacI^{q} lacZΔM15], est employée pour la construction de plasmides, et pour l'amplification et l'isolement de plasmides. Elle est cultivée sur le milieu LB.

Les plasmides mis en oeuvre sont les suivants :

Le vecteur pGAD10, fourni par Clontech® permet l'expression chez la levure d'une protéine de fusion entre PAK1 (domaine CRIB) ou c-Raf1 (domaine RBD) et le domaine transactivateur de GAL4 (protéine PAK1-TA ou c-Raf1-TA).

Le vecteur pGBT9, fourni par Clontech® permet l'expression chez la levure d'une protéine de fusion entre Ras, Rac ou Cdc42 et le domaine d'interaction à l'ADN de GAL4 (protéines Ras-BD ou Rac-BD ou Cdc42-BD).

Un vecteur qui permet l'expression dans la levure de GRF1 (tout ou parties).

### Transformation de la levure par un plasmide

Le levure CL9 est rendue compétente par un traitement au LiAC/PEG d'après la méthode décrite par Gietz *et al,* (49). Elle est ensuite transformée par 1µg de chacun des plasmides permettant l'expression des protéines de fusion et GRF1 qui composent le système double-hybride plus un. L'expression de celles-ci conduit à une sensibilité de la souche au cycloheximide.

Un produit interférant avec l'activation par GRF1 des protéines de fusion Ras-BD, Rac-BD ou Cdc42-BD du système permettra la croissance de la levure sur ce type de milieu. Pour faire un criblage, on étale le levure à la surface d'un milieu sélectif contenant 10µg/ml de cycloheximide. Des gouttes de composés chimiques sont appliquées directement à la surface de la boîte. Les produits sélectionnés sont ceux qui donnent une auréole de croissance autour du dépôt.

Le criblage est fait dans une levure perméabilisée selon les techniques décrites précédemment.

La spécificité des produits pour l'activation par GRF1 des protéines Ras-BD ou Rac-BD ou Cdc42-BD sera validée par un des autres tests donnés en exemple.

D'autres techniques de révélation d'un système double-hybride plus un peuvent être mis en oeuvre.

En inhibant l'interaction protéine-protéine, les molécules actives peuvent être révélées par la diminution de l'expression d'un gène rapporteur. Celle-ci pouvant, selon le cas, être mise en évidence par un test colorimétrique, fluorimétrique ou enzymatique. L'inhibition de la croissance peut être mesurée par l'utilisation d'un milieu sélectif (ex : milieu au fluorate pour l'utilisation du gène rapporteur URA3 ou milieu à la canavanine pour CAN1).

A noter que dans le cas d'interactions faibles ou très fugaces entre protéines, il est possible d'envisager d'introduire des mutations dans celles-ci afin de rendre l'interaction plus forte (52).

Pour la mise en oeuvre de ces derniers exemples l'homme du métier peut se référer aux brevets US 5,283,173 **,** US 5,468,614**,** US 5,525,490**,** US 5,580,736 **et** US 5,885,779**.**

### Exemple 5 : Utilisation de la protéine GRF1 pour la réalisation de cribles de composés par mesure de l'interaction entre GRF1 et les sous-unités béta-gamma des protéines G hétéro-trimériques

Il existe une interaction physique entre le domaine PH de GRF1 et les sous-unités béta-gamma des protéines G hétéro-trimériques (7-9). Le système double-hybride dans la levure (tel que décrit dans l'exemple 4) semble être l'outil adapté pour la réalisation d'un crible permettant la mise en évidence de molécules capables de perturber cette interaction. Il pourrait utiliser soit la protéine GRF 1 entière, soit les domaines PH, soit l'enchaînement de domaines PH-DH-PH tel qu'il se trouve naturellement dans la protéine GRF1.

### Exemple 6 : Utilisation de GRF1 dans un test de criblage cellulaire pour inhibiteurs de GRF1 mettant en oeuvre des cellules transfectées.

D'après la littérature, la surexpression des parties catalytiques de GRF1 confère à la cellule transfectée le phénotype transformé (46, 47).

Le test de criblage pour des inhibiteurs de GRF1 utilise les clones stables de cellules transfectées par *grf1*, pour lesquels les présents inventeurs ont pu montrer une surexpression du transgène par western-blot.

La surexpression de GRF1 confère aux cellules la capacité à cloner en agar qui est vérifié selon le protocole suivant :

Les cellules sont comptées et resuspendues dans du milieu complet sans rouge de phénol et à une concentration de 2,5. 10⁵ cellules par ml.

Dans les puits de plaques à 96 puits, on coule successivement :
75 µl de " Bottom layer " (couche de base) contenant 50% de milieu de culture 2X sans rouge de phénol et 50% d'agarose ((1,2%, " low melting point " (à bas point de fusion) comme par exemple SIGMA (A-6560) type VII préparée dans de l'eau et autoclavée)),
75 µl de " top layer " préparés à partir du mélange suivant :
   - 25 ml de milieu 1.67X sans rouge de phénol (500 ml de milieu 2X sans rouge de phénol, 100 ml de sérum de veau foetal, 2X glutamine et 2X penicilline/streptomycine), 100 ml d'eau stérile), 10 ml de suspension cellulaire, 15 ml d'agarose (1,2%, " low melting point " comme par exemple SIGMA (A-6560) type VII préparée dans de l'eau et autoclavée).

Une fois l'agar solidifié, les molécules à tester sont diluées dans du milieu 1X aux concentrations qui conviennent et 75 µl sont ajoutés sur les couches d'agar dans les puits.

Les cellules sont comptées après deux semaines par une coloration à la calcéine : La Calcéine AM (Molecular Probes C-1430, 4 mM DMSO) est diluée à 10 µM dans du HBSS et conservée à -20°C. 25 µl de calcéine AM sont ajoutés dans chaque puits et les cellules sont laissées à l'incubateur (37°C) pendant au moins une heure. La plaque est lue dans un appareil C0

Un inhibiteur de GRF1 sera révélé par la diminution du nombre de clones en agar. Toute cellule mammifère immortalisée et transfectée par un vecteur d'expression eucaryote (plasmide) permettant l'expression de tout ou partie (domaine CDC25-like ou domaine DH) de *grf1*, humain ou murin peut aussi être utilisée. Ce vecteur d'expression confère en même temps le phénotype résistant à un agent de sélection comme l'hygromycine, la néomycine, la zéocyne ou autres et permet de cette façon de sélectionner puis de cloner (par FACS ou par dilution limite) les cellules transfectées et exprimant le transgène.

**Tableau : localisation des domaines fonctionnels des protéines GRF 1 mammifères**

| | | | |
|---|---|---|---|
| espèce | Homme | Souris | Rat |
| Taille protéine | 1275 | 1262 | 1244 |
| Pleckstrin homology #1 (PH) | 22-129 | 22-130 | 22-129 |
| Dbl homology (DH) domaine d'échange sur Rac | 244-455 | 248-459 | 244-455 |
| Pleckstrin homology #2 (PH) | 456-590 | 460-588 | 456-582 |
| CDC25 homology domain domaine d'échange sur Ras | 1045-1272 | 1032-1259 | 1014-1241 |

### Références bibliographiques

1- Hall, A., Rho GTPases and the actin cytoskeleton. Science, 279, 509-14 (1998).
2- Kiyono, M., et al., G protein βγ subunit-dependent Rac-guanine nucleotide exchange activity of Ras-GRF1/CDC25Mm. Proc. Natl. Sci. USA, 96, 4826-4831 (1999).
3- Farnsworth, C.L., et al., Calcium activation of Ras mediated by neuronal exchange factor Ras-GRF. Nature, 376, 524-527 (1995).
4- Freshney, NW., et al., Activation of the exchange factor Ras-GRF by calcium requires an intact Dbl homology domain. FEBS Lett, 407, 111-5 (1997).
5- Buchsbaum, R., et al., The N-terminal pleckstrin, coiled-coil, and IQ domains of the exchange factor Ras-GRF act cooperatively to facilitate activation by calcium. Mol Cell Biol, 16, 4888-96 (1996).
6- Schweighoffer, F., et al., Identification of a human guanine nucleotide-releasing factor (H-GRF55) specific for Ras proteins. Oncogene, 8, 1477-1485 (1993).
7- Sawai, T., et al., Interaction between Pleckstrin homology domains and G protein betagamma-subunits: analyses of kinetic parameters by a biosensor-based method. Biol Pharm Bull, 22, 229-33 (1999).
8- Shaw,G., The pleckstrin homology domain: an intriguing multifunctional protein module. Bioessays, 18, 35-46 (1996).
9- Touhara, K., et al., Binding of G protein beta gamma-subunits to pleckstrin homology domains. J Biol Chem, 269, 10217-20 (1994).
10- Martegani, E., et al., Cloning by functional complementation of a mouse cDNA encoding a homologue of CDC25, a Saccharomyces cerevisiae RAS activator. EMBO J , 11, 2151-2157 (1992).
11- Shou, C., et al., Molecular cloning of cDNAs encoding a guanine-nucleotide-releasing factor for Ras p21. Nature, 358, 351-354 (1992).
12- Wei, W., et al., Localization of the cellular expression pattern of CDC25NEF and ras in the juvenile rat brain. Molecular brain research, 19, 339-344 (1993).
13- Brambilla, R., et al., A role for the Ras signalling pathway in synaptic transmission and long-term memory. Nature, 390, 281-286 (1997).
14- Itier, JM., et al., Imprinted gene in postnatal role. Nature, 393, 125-126 (1998).
15- Zippel, R, et al., Ras-GRF, the activator of Ras, is expressed preferentially in mature neurons of the central nervous system. Molecular brain research, 48, 140-144 (1997).
16- Plass, C., et al., Identification of GRF1 on mouse chromosome 9 as an imprinted gene by RLGS-M. Nature genetics, 14, 106-109 (1996).
17- Shou, C., et al., Differential response of the Ras exchange factor, Ras-GRF to tyrosine kinase and G protein mediated signals. Oncogene, 10, 1887-1893 (1995).
18- Mattingly, RR. and Macara, IG., Phosphorylation-dependent activation of the Ras-GRF/CDC25Mm exchange factor by muscarinic receptors and G-protein βγ subunits. Nature, 382, 268-272 (1996).
19- Zippel, R., et al., The brain specific Ras exchange factor CDC25Mm: modulation of its activity through Gi-protein- mediated signals. Oncogene, 12, 2697-2703 (1996).
20- Friedman, JM., Halaas, JL., Leptin and the regulation of body weight in mammals. Nature, 395, 763-70 (1998).
21- Schwartz, MW., et al., Identification of targets of leptin action in rat hypothalamus. J Clin Invest, 98, 1101-6 (1996).
22- Kristensen, P., et al., Hypothalamic CART is a new anorectic peptide regulated by leptin. Nature, 393, 72-6 (1998).
23- Krude, H., et al., Severe early-onset obesity, adrenal insufficiency and red hair pigmentation caused by POMC mutations in humans. Nat Genet, 19, 155-7 (1998).
24- Yeo, GS., et al., A frameshift mutation in MC4R associated with dominantly inherited human obesity. Nat Genet, 20, 111-2 (1998).
25- Vaisse, C., et al., A frameshift mutation in human MC4R is associated with a dominant form of obesity. Nat Genet, 20, 113-4 (1998).
26- Sierra-Honigmann, MR., et al., Biological action of leptin as an angiogenic factor. Science, 281, 1683-6 (1998).
27- Bouloumie, A., et al., Leptin, the product of Ob gene, promotes angiogenesis. Circ Res, 83, 1059-66 (1998).
28- Chehab, FF., et al., Correction of the sterility defect in homozygous obese female mice by treatment with the human recombinant leptin. Nat Genet, 12, 318-20 (1996).
29- Barash, IA., et al., Leptin is a metabolic signal to the reproductive system. Endocrinology, 137, 3144-7 (1996).
30- Chehab, FF., et al., Early onset of reproductive function in normal female mice treated with leptin. Science, 275, 88-90 (1997).
31- Strobel, A., et al., A leptin missense mutation associated with hypogonadism and morbid obesity. Nat Genet, 18, 213-5 (1998).
32- Clement, K., et al., A mutation in the human leptin receptor gene causes obesity and pituitary dysfunction. Nature, 392, 398-401 (1998).
33- Lord, GM., et al., Leptin modulates the T-cell immune response and reverses starvation-induced immunosuppression. Nature, 394, 897-901 (1998).
34- Ducy P. et al., Leptin inhibits bone formation through a hypothalamic relay : a central control of bone mass. Cell, 100, 197-207, 2000.
35- Thiele, TE., et al., Ethanol consumption and resistance are inversely related to neuropeptide Y levels. Nature, 396, 366-9 (1998).
36- Tamura, A., et al., Focal cerebral ischemia in the rat :1. Description of technique and early neuropathological consequences following middle cerebral artery occlusion. J Cereb Blood Flow Metab, 1, 53-60 (1981).
37- Bederson, JA., et al., Rat middle cerebral artery occlusion : evaluation of the model and development of a neurologic examination. Stroke, 17, 472-476 (1986).
38- Chatton, B., et al., Eukaryotic GST fusion vector for the study of protein-protein associations in vivo: application to interaction of ATFa with Jun and Fos. Biotechniques, 18, 142-5 (1995).
39- Lowe N et al., Delineation of the Cdc42/Rac-binding Domain of p21-Activated Kinase. Biochemistry, 37, 7885-7891, (1998).
40- Methods Enzymol., Guide to yeast genetics and molecular biology, 194, 1-863 (1991).
41- Biotechnology, Yeast genetics engineering, 13, 1-354 (1989).
42- Methods in molecular biology, Yeast protocols, 53, 1-433 (1996).
43- Molecular Genetics of Yeast : A Practical Approach. Edited by John R. Johnston, 1-300 (1994).
44- The Yeast Two-Hybrid System. Paul L. Bartel and Stanley Fields, 1-356 (1997).
45- Methods in Yeast Genetics : A Laboratory Course Manual (1997).
46- Chevallier-Multon MC et al. Saccharomyces cerevisiae CDC25 (1028-1589) is a guanine nucleotide releasing factor for mammalian Ras proteins and is oncogenic in NIH3T3 cells. J. Biol. chem. 268, pp11113-11119, 1993.
47- Barlat I et al., The Saccharomyces cerevisiae gene product SDC25 C-domain functions as an oncoprotein in NIH3T3 cells. Oncogene, 8, 215-218, 1993
48- Han, J., et al., Lck regulates Vav activation of members of the Rho family of GTPases.Mol Cell Biol, 17, pp1346-1353, 1997.
49- Gietz, R.D., R. H. Schiestl, A. R. Willems, and R. A. Woods. Studies on the transformation of intact yeast cells by LiAC/SS-DNA/PEG procedure. Yeast, 11 : 355-360.1995.
50- Mosteller, RD., et al., Analysis of interaction between Ras and CDC25 guanine nucleotide exchange factor using yeast GAL4 two-hybrid system. Methods Enzymol. 255, 135-48 (1995).
51- Leanna, C.A., Hannink M. The reverse two-hybrid system : a genetic scheme for selection against specific protein/protein interactions. Nucleic.Acids.Research. 96, 3341-3347. 1996.
52- Kamada, S., et al., A cloning method for caspase substrates that uses the yeast two-hybrid system: cloning of the antiapoptotic gene gelsolin. Proc Natl Acad Sci U S A , 95, 8532-7 (1998).
53- Capon,D.J., Chen,E.Y., Levinson,A.D., Seeburg,P.H. and Goeddel,D.V. Complete nucleotide sequences of the T24 human bladder carcinoma oncogene and its normal homologue, Nature 302 (5903), 33-37 (1983)
54- McGrath,J.P., Capon,D.J., Smith,D.H., Chen,E.Y., Seeburg,P.H., Goeddel,D.V. and Levinson,A.D. Structure and organization of the human Ki-ras proto-oncogene and a related processed pseudogene.Nature 304 (5926), 501-506 (1983)
55- Taparowsky,E., Shimizu,K., Goldfarb,M. and Wigler,M .Structure and activation of the human N-ras gene. Cell 34 (2), 581-586 (1983)
56- Didsbury,J., Weber,R.F., Bokoch,G.M., Evans,T. and Snyderman,R. rac, a novel ras-related family of proteins that are botulinum toxin substrates, J. Biol. Chem. 264 (28), 16378-16382 (1989)
57- Polakis,P.G., Weber,R.F., Nevins,B., Didsbury,J.R., Evans,T. And Snyderman,R., Identification of the ral and racl gene products, low molecular mass GTP-binding proteins from human platelets. J. Biol. Chem. 264 (28), 16383-16389 (1989)
58- Drivas,G.T., Shih,A., Coutavas,E., Rush,M.G. and D'Eustachio,P. Characterization of four novel ras-like genes expressed in a human teratocarcinoma cell line. Mol. Cell. Biol. 10 (4), 1793-1798 (1990)
59- Shinjo,K., Koland,J.G., Hart,M.J., Narasimhan,V., Johnson,D.I., Evans,T. and Cerione,R.A. Molecular cloning of the gene for the human placental GTP-binding protein Gp (G25K): identification of this GTP-binding protein as the human homolog of the yeast cell-division-cycle protein CDC42. Proc. Natl. Acad. Sci. U.S.A. 87 (24), 9853-9857 (1990)
60- Brown,J.L., Stowers,L., Baer,M., Trejo,J., Coughlin,S. and Chant,J. Human Ste20 homologue hPAK1 links GTPases to the JNK MAP kinase pathway. Curr. Biol. 6 (5), 598-605 (1996)
61- Bekri S, Adelaide J, Merscher S, Grosgeorge J, Caroli-Bosc F, Perucca-Lostanlen D, Kelley PM, Pebusque MJ, Theillet C, Birnbaum D and Gaudray P. Detailed map of a region commonly amplified at 11q13-->q14 in human breast carcinoma Cytogenet. Cell Genet. 79 (1-2), 125-131 (1997)
62- Sanders,L.C., Matsumura,F., Bokoch,G.M. and de Lanerolle,P. Inhibition of myosin light chain kinase by p21-activated kinase. Science 283 (5410), 2083-2085 (1999)
63- Sells,M.A., Boyd,J.T. and Chernoff,J. p21-activated kinase 1 (Pak1) regulates cell motility in mammalian fibroblasts. J. Cell Biol. 145 (4), 837-849 (1999)
64- Bagrodia,S. and Cerione,R.A. Pak to the future.Trends Cell Biol. 9 (9), 350-355 (1999)
65- Bonner,T.I., Oppermann,H., Seeburg,P., Kerby,S.B., Gunnell,M.A., Young,A.C. and Rapp,U.R. The complete coding sequence of the human raf oncogene and the corresponding structure of the c-raf-1 gene Nucleic Acids Res. 14 (2), 1009-1015 (1986)
66- Nassar,N., Horn,G., Herrmann,C., Scherer,A., McCormick,F. And Wittinghofer,A. The 2.2 A crystal structure of the Ras-binding domain of the serine/threonine kinase c-Raf1 in complex with RaplA and a GTP analogue Nature 375 (6532), 554-560 (1995)
67- Nassar,N., Horn,G., Herrmann,C., Block,C., Janknecht,R. And Wittinghofer,A. Ras/Rap effector specificity determined by charge reversal Nat. Struct. Biol. 3 (8), 723-729 (1996)
68- Emerson,S.D., Madison,V.S., Palermo,R.E., Waugh,D.S., Scheffler,J.E., Tsao,K.L., Kiefer,S.E., Liu,S.P. and Fry,D.C. Solution structure of the Ras-binding domain of c-Raf-1 and identification of its Ras interaction surface Biochemistry 34 (21), 6911-6918(1995)
69- Mott,H.R., Carpenter,J.W., Zhong,S., Ghosh,S., Bell,R.M. and Campbell,S.L. The solution structure of the Raf-1 cysteine-rich domain: a novel ras and phospholipid binding site Proc. Natl. Acad. Sci. U.S.A. 93 (16), 8312-8317 (1996)
70-Vojtek, A.B., S.M. Hollenberg, and J.A.Cooper.. Mammalian Ras interacts directly with the serine/threonine kinase Raf. Cell, 74 : 205-214. (1993).

## Revendications

1. Utilisation d'une protéine présentant une identité d'au moins 85% avec une protéine Guanine Nucleotide Releasing Factor 1 (GRF1) on de cellules exprimant une telle protéine, dans une méthode de détection de composés destinés à la prévention et / ou au traitement de pathologies ou de désordres du système nerveux central impliquant une mort neuronale ou liés à l'obésité.

2. Utilisation selon la revendication 1, **caractérisée en ce que** ladite méthode de détection comporte une étape de mesure de la phosphorylation de la protéine GRF1.

3. Utilisation selon la revendication 1, **caractérisée en ce que** ladite méthode comprend une étape de mesure de l'activité d'échange de la protéine GRF1 sur des protéines de la famille des petites protéines G, notamment Ras, Rac et Cdc42.

4. Utilisation selon la revendication 1, **caractérisée en ce que** ladite méthode comprend une étape de mesure de l'interaction entre la protéine GRF1 et les sous-unités béta-gamma des protéines G hétérotrimériques.

5. Utilisation selon la revendication 1, **caractérisée en ce que** ladite méthode de détection comporte une étape de mise en contact des composés à cribler avec des levures transformées par *grf1*.

6. Utilisation selon la revendication 5, **caractérisée en ce que** ladite méthode de détection comporte une étape de mise en contact des composés à cribler avec des levures transformées par *grf1* et dont les gènes CDC25 et/ou CDC24 ont été inactivés ou mutés.

7. Utilisation selon la revendication 1, **caractérisée en ce que** ladite méthode de détection comporte une étape de mise en contact des composés à cribler avec des levures exprimant une première protéine hybride constituée par une protéine de fusion entre Ras, CDC42Hs ou Rac et un domaine d'interaction à l'ADN, et une deuxième protéine hybride constituée par une protéine de fusion entre tout ou partie de GRF 1 et un domaine transactivateur.

8. Utilisation selon la revendication 1, **caractérisée en ce que** ladite méthode de détection comporte une étape de mise en contact des composés à cribler avec des levures exprimant une proteine présentant une identité d'au moins 85% avec une protéine GRF1, PAK1 fusionnée avec un domaine transactivateur, et Rac ou CDC42Hs fusionnée avec un domaine d'interaction à l'ADN.

9. Utilisation selon la revendication 1, **caractérisée en ce que** ladite méthode de détection comporte une étape de mise en contact des composés à cribler avec des levures exprimant une proteine présentant une identité d'au moins 85% avec une protéine GRF1, Raf1 fusionnée avec un domaine transactivateur et Ras fusionnée avec un domaine d'interaction à l'ADN.

10. Utilisation selon l'une quelconque des revendications précédentes **caractérisée en ce que** la pathologie est l'ischémie cérébrale, la maladie de Parkinson ou la maladie d'Alzheimer.

11. Procédé de criblage ou de détection de composés destinés à la prévention et / ou au traitement de pathologies du système nerveux central impliquant une mort neuronale comprenant les étapes consistant à
(i) mettre en culture des cellules constituées par des cellules exprimant la protéine GRF1 en présence d'un substrat phosphoryle marqué d'un composé à tester,
(ii) lyser lesdites cellules, et
(iii) mesurer la quantité de protéine GRF 1 marquée.

12. Procédé de criblage ou de détection de composés destinés à la prévention et / ou au traitement de pathologies du système nerveux central impliquant une mort neuronale comprenant les étapes préalables consistant à :
(i) mettre en culture des cellules constituées par des cellules exprimant la protéine GRF1en présence d'un composé à tester,
(ii) lyser lesdites cellules, et
(iii) mesurer la quantité de GRF1 phosphorylée à l'aide d'un anticorps spécifique d'un acide aminé phosphorylé.

13. Procédé de criblage ou de détection de composés destinés à la prévention et / ou au traitement de pathologies du système nerveux central impliquant une mort neuronale comprenant les étapes consistant à:
(i) mettre en présence la protéine GRF 1, tout ou partie d'une protéine de la famille des petites protéines G chargée en GDP ou en GTP marqué, et un composé à tester,
(ii) ajouter respectivement du GTP ou du GDP non marqué, et
(iii) mesurer la quantité de protéine G marquée.

14. Procédé de détection par criblage cellulaire et *in vitro* de composés destinés à la prévention et / ou au traitement de pathologies du système nerveux central impliquant une mort neuronale comprenant les étapes consistant à
(i) transfecter des cellules mammifères immortalisées par un vecteur exprimant un transgène grf1 et conférant auxdites cellules une résistance à un agent de sélection
(ii) sélectionner et cloner lesdites cellules transfectées et exprimant le transgène et les cloner dans un support de croissance en agar,
(iii) ajouter lesdits composés à tester à une suspension desdites cellules, et
(iv) détecter lesdits composés inhibiteurs de GRF1 par diminution du nombre de clones.

## Claims

1. Use of a protein exhibiting at least 85% identity with a Guanine Nucleotide Releasing Factor 1 (GRF1) protein, or of cells expressing such a protein, in Method for detecting compounds intended for the prevention and/or treatment of pathological conditions or of disorders of the central nervous system involving neuronal death, or associated with obesity.

2. Use according to Claim 1, **characterized in that** said detection method comprises a step consisting of measuring phosphorylation of the GRF1 protein.

3. Use according to Claim 1, **characterized in that** said method comprises a step consisting of measuring exchange activity of the GRF1 protein on proteins of the family of small G proteins, in particular Ras, Rac and Cdc42.

4. Use according to Claim 1, **characterized in that** said method comprises a step consisting of measuring the interaction between the GRF1 protein and the beta-gamma subunits of the heterotrimeric G proteins.

5. Use according to Claim 1, **characterized in that** said detection method comprises a step consisting of bringing the compounds to be screened into contact with yeasts transformed with *grf1.*

6. Use according to Claim 5, **characterized in that** said detection method comprises a step consisting of bringing the compounds to be screened into contact with yeast transformed with *grf1* and in which the CDC25 and/or CDC24 genes have been inactivated or mutated.

7. Use according to Claim 1, **characterized in that** said detection method comprises a step consisting of bringing the compounds to be screened into contact with yeast expressing a first hybrid protein consisting of a fusion protein between Ras, CDC42Hs or Rac and a DNA-interacting domain, and a second hybrid protein consisting of a fusion protein between all or part of GRF1 and a transactivating domain.

8. Use according to Claim 1, **characterized in that** said detection method comprises a step consisting of bringing the compounds to be screened into contact with yeast expressing a protein exhibiting at least 85% identity with GRF1 protein, a PAK1 protein fused with a transactivating domain, and a Rac protein or a CDC42Hs protein fused with a DNA-interacting domain.

9. Use according to Claim 1, **characterized in that** said detection method comprises a step consisting of bringing the compounds to be screened into contact with yeast expressing a protein exhibiting at least 85% identity with a GRF1 protein, a Raf1 protein fused with a transactivating domain and a Ras protein fused with a DNA-interacting domain.

10. Use according to any one of the preceding claims, **characterized in that** the pathological condition is cerebral ischemia, Parkinson's disease or Alzheimer's disease.

11. Method for screening or for detecting compounds intended for the prevention and/or treatment of pathological conditions of the central nervous system involving neuronal death, comprising the steps consisting in:
(i) culturing cells consisting of cells expressing the GRF1 protein in the presence of a labeled phosphorylated substrate and of a test compound,
(ii) lyzing said cells, and
(iii) measuring the amount of labeled GRF1 protein.

12. Method for screening or for detecting compounds intended for the prevention and/or treatment of pathological conditions of the central nervous system involving neuronal death, comprising the prior steps consisting in:
(i) culturing cells consisting of cells expressing the GRF1 protein in the presence of a test compound,
(ii) lyzing said cells, and
(iii) measuring the amount of phosphorylated GRF1 using an antibody specific for a phosphorylated amino acid.

13. Method for screening or for detecting compounds intended for the prevention and/or treatment of pathological conditions of the central nervous system involving neuronal death, comprising the steps consisting in:
(i)bringing into contact the GRF1 protein, all or part of a protein of the small G protein family loaded with labeled GDP or GTP, and a test compound,
(ii)adding respectively unlabeled GTP or GDP, and
(iii)measuring the amount of labeled G protein.

14. Method for detecting, by cell screening and *in vitro,* compounds intended for the prevention and/or treatment of pathological conditions of the central nervous system involving neuronal death, comprising the steps consisting in:
(i) transfecting immortalized mammalian cells with a vector expressing a grf1 transgene and imparting on said cells resistance to a selection agent,
(ii) selecting and cloning said cells transfected and expressing the transgene and cloning them in an agar growth support,
(iii) adding said test compounds to a suspension of said cells, and
(iv) detecting said GRF1-inhibiting compounds by a decrease in the number of clones.

## Patentansprüche

1. Verwendung eines Proteins mit mindestens 85% Identität zu dem Protein Guanin Nucleotide Releasing Factor 1 (GRF1) oder von Zellen, die solch ein Protein exprimieren, in einem Verfahren zum Nachweisen von Verbindungen für die Vorbeugung und/oder Behandlung von Pathologien oder Störungen des Zentralnervensystems, die mit dem Absterben von Neuronen einhergehen oder mit Fettleibigkeit assoziiert sind.

2. Verwendung nach Anspruch 1, **dadurch gekennzeichnet, daß** dieses Nachweisverfahren einen Schritt umfaßt, bei dem das Protein GRF1 phosphoryliert wird.

3. Verwendung nach Anspruch 1, **dadurch gekennzeichnet, daß** dieses Verfahren einen Schritt umfaßt, bei dem man die Aktivität des Austausches des Proteins GRF1 mit Proteinen der Familie der kleinen G-Proteine, insbesondere Ras, Rac und Cdc42, bestimmt.

4. Verwendung nach Anspruch 1, **dadurch gekennzeichnet, daß** dieses Verfahren einen Schritt umfaßt, bei dem man die Interaktion zwischen dem Protein GRF1 und den beta-gamma-Untereinheiten von heterotrimeren G-Proteinen bestimmt.

5. Verwendung nach Anspruch 1, **dadurch gekennzeichnet, daß** dieses Nachweisverfahren einen Schritt umfaßt, bei dem man zu durchmusternde Verbindungen mit *grf1*-transformierten Hefen in Kontakt bringt.

6. Verwendung nach Anspruch 5, **dadurch gekennzeichnet, daß** dieses Nachweisverfahren einen Schritt umfaßt, bei dem man zu durchmusternde Verbindungen mit *grf1*-transformierten Hefen, deren CDC25- und/oder CDC24-Gen(e) inaktiviert oder mutiert worden sind, in Kontakt bringt.

7. Verwendung nach Anspruch 1, **dadurch gekennzeichnet, daß** dieses Nachweisverfahren einen Schritt umfaßt, bei dem man zu durchmusternde Verbindungen mit Hefen in Kontakt bringt, die ein erstes Hybridprotein, das aus einem Fusionsprotein zwischen Ras, CDC42Hs oder Rac und einer DNA-Interaktionsdomäne besteht, sowie ein zweites Hybridprotein, das aus einem Fusionsprotein zwischen dem gesamten GRF1 oder einem Teil von GRF1 und einer Transaktivatordomäne besteht, exprimieren.

8. Verwendung nach Anspruch 1, **dadurch gekennzeichnet, daß** dieses Nachweisverfahren einen Schritt umfaßt, bei dem man zu durchmusternde Verbindungen mit Hefen in Kontakt bringt, die ein Protein mit mindestens 85% Identität zu einem Protein GRF1, PAK1 in Fusion mit einer Transaktivatordomäne, sowie Rac oder CDC42Hs in Fusion mit einer DNA-Interaktionsdomäne exprimieren.

9. Verwendung nach Anspruch 1, **dadurch gekennzeichnet, daß** dieses Nachweisverfahren einen Schritt umfaßt, bei dem man zu durchmusternde Verbindungen mit Hefen in Kontakt bringt, die ein Protein mit mindestens 85% Identität zu einem Protein GRF1, Raf1 in Fusion mit einer Transaktivatordomäne, sowie Ras in Fusion mit einer DNA-Interaktionsdomäne exprimieren.

10. Verwendung nach einem der vorhergehenden Ansprüche, **dadurch gekennzeichnet, daß** es sich bei der Pathologie um Hirnischämie, Morbus Parkinson oder Morbus Alzheimer handelt.

11. Verfahren zum Durchmustern oder Nachweisen von Verbindungen für die Vorbeugung und/oder Behandlung von Pathologien des Zentralnervensystems, die mit dem Absterben von Neuronen einhergehen, umfassend die folgenden Schritte:
(i) Kultivieren von Zellen, die aus Zellen, die das Protein GRF1 exprimieren, bestehen, in Gegenwart eines markierten phosphorylierten Substrats und einer Testverbindung,
(ii) Lysieren dieser Zellen, sowie
(iii)Bestimmen der Menge des markierten Proteins GRF1.

12. Verfahren zum Durchmustern oder Nachweisen von Verbindungen für die Vorbeugung und/oder Behandlung von Pathologien des Zentralnervensystems, die mit dem Absterben von Neuronen einhergehen, bei dem man zuvor die folgenden Schritte durchführt:
(i) Kultivieren von Zellen, die aus Zellen, die das Protein GRF1 exprimieren, bestehen, in Gegenwart einer Testverbindung,
(ii) Lysieren dieser Zellen, sowie
(iii)Bestimmen der Menge an phosphoryliertem GRF1 mit einem Antikörper, der für eine phosphorylierte Aminosäure spezifisch ist.

13. Verfahren zum Durchmustern oder Nachweisen von Verbindungen für die Vorbeugung und/oder Behandlung von Pathologien des Zentralnervensystems, die mit dem Absterben von Neuronen einhergehen, umfassend die folgenden Schritte:
(i) Vorlegen des Proteins GRF1, eines ganzen Proteins bzw. eines Teils eines Proteins der Familie der kleinen G-Proteine, das mit markiertem GDP oder GTP beaufschlagt wurde, sowie einer Testverbindung,
(ii) Versetzen mit unmarkiertem GTP bzw. GDP sowie
(iii) Bestimmen der Menge an markiertem Protein G.

14. Verfahren zum Nachweisen von Verbindungen für die Vorbeugung und/oder Behandlung von Pathologien des Zentralnervensystems, die mit dem Absterben von Neuronen einhergehen, mittels in-vitro-Durchmustern von Zellen umfassend die folgenden Schritte:
(i) Transfektion von immortalisierten Säugetierzellen mit einem Vektor, der ein grf1-Transgen exprimiert und diesen Zellen eine Resistenz gegen ein Selektionsmittel verleiht,
(ii) Selektion und Klonieren dieser transfizierten Zellen, die das Transgen exprimieren, und Klonieren in einem Agarwachstumsmedium,
(iii) Versetzen der Testverbindungen mit einer Suspension dieser Zellen, sowie
(iv) Nachweisen der GRF1-Hemmer-Verbindungen durch zahlenmäßige Verringerung der Klone.


### LISTE DE SEQUENCES

<110> AVENTIS PHARMA SA
<120> Utilisation de la protéine GRF1
<130> GRF1
<140>
   <141>
<160> 3
<170> PatentIn Ver. 2.1
<210> 1
   <211> 1275
   <212> PRT
   <213> Homo sapiens
<400> 1
<210> 2
   <211> 1262
   <212> PRT
   <213> Mus musculus
<400> 2
   Met Gln Lys Ala Ile Arg Leu Asn Asp Gly His Val Val Thr Leu Gly
   1 5 10 15
   Leu Leu Ala Gln Lys Asp Gly Thr Arg Lys Gly Tyr Leu Ser Lys Arg
   20 25 30
   Ser Ala Asp Asn Pro Lys Trp Gln Thr Lys Trp Phe Ala Leu Leu Gln
   35 40 45
   Asn Leu Leu Phe Tyr Phe Glu Ser Asp Ser Ser Pro Arg Pro Ser Gly
   50 55 60
   Leu Tyr Leu Leu Glu Gly Ser Ile Cys Lys Arg Ala Pro Ser Pro Lys
   65 70 75 80
   Arg Gly Thr Ser Ser Lys Glu Ser Gly Glu Lys Gln Gln His Tyr Phe
   85 90 95
   Thr Val Asn Phe Ser Asn Asp Ser Gln Lys Thr Leu Glu Leu Arg Thr
   100 105 110
   Glu Asp Ala Lys Asp Cys Asp Glu Trp Val Ala Ala Ile Ala Arg Ala
   115 120 125
   Ser Tyr Lys Ile Leu Ala Thr Glu His Glu Ala Leu Met Gln Lys Tyr
   130 135 140
   Leu His Leu Leu Gln Val Val Glu Thr Glu Lys Thr Val Ala Lys Gln
   145 150 155 160
   Leu Arg Gln Gln Leu Glu Asp Gly Glu Val Glu Ile Glu Arg Leu Lys
   165 170 175
   Thr Glu Val Thr Ile Thr Asn Leu Ile Lys Asp Asn Asp Arg Ile Gln
   180 185 190
   Ser Ser Asn Lys Ala Gly Ser Ala Asp Asp Glu Asp Ser Asp Ile Lys
   195 200 205
   Lys Ile Lys Lys Val Gln Ser Phe Leu Arg Gly Trp Leu Cys Arg Arg
   210 215 220
   Lys Trp Lys Asn Ile Ile Gln Asp Tyr Ile Arg Ser Pro His Ala Asp
   225 230 235 240
   Ser Met Arg Lys Arg Asn Gln Val Val Phe Ser Met Leu Glu Ala Glu
   245 250 255
   Ala Glu Tyr Val Gln Gln Leu His Ile Leu Val Asn Asn Phe Leu Arg
   260 265 270
   Pro Leu Arg Met Ala Ala Ser Ser Lys Lys Pro Pro Ile Thr His Asp
   275 280 285
   Asp Val Ser Ser Ile Phe Leu Asn Ser Glu Thr Ile Met Phe Leu His
   290 295 300
   Gln Ile Phe Tyr Gln Gly Leu Lys Ala Arg Ile Ser Ser Trp Pro Thr
   305 310 315 320
   Leu Val Leu Ala Asp Leu Phe Asp Ile Leu Leu Pro Met Leu Asn Ile
   325 330 335
   Tyr Gln Glu Phe Val Arg Asn His Gln Tyr Ser Leu Gln Ile Leu Ala
   340 345 350
   His Cys Lys Gln Asn Arg Asp Phe Asp Lys Leu Leu Lys Gln Tyr Glu
   355 360 365
   Ala Lys Pro Asp Cys Glu Glu Arg Thr Leu Glu Thr Phe Leu Thr Tyr
   370 375 380
   Pro Met Phe Gln Ile Pro Arg Tyr Ile Leu Thr Leu His Glu Leu Leu
   385 390 395 400
   Ala His Thr Pro His Glu His Val Glu Arg Asn Ser Leu Asp Tyr Ala
   405 410 415
   Lys Ser Lys Leu Glu Glu Leu Ser Arg Ile Met His Asp Glu Val Ser
   420 425 430
   Glu Thr Glu Asn Ile Arg Lys Asn Leu Ala Ile Glu Arg Met Ile Thr
   435 440 445
   Glu Gly Cys Glu Ile Leu Leu Asp Thr Ser Gln Thr Phe Val Arg Gln
   450 455 460
   Gly Ser Leu Met Gln Met Ser Leu Ser Glu Lys Ser Lys Ser Ser Arg
   465 470 475 480
   Gly Arg Leu Gly Ser Leu Ser Thr Lys Lys Glu Gly Glu Arg Gln Cys
   485 490 495
   Phe Leu Phe Ser Lys His Leu Ile Ile Cys Thr Arg Gly Ser Gly Gly
   500 505 510
   Lys Leu His Leu Thr Lys Asn Gly Val Ile Ser Leu Ile Asp Cys Thr
   515 520 525
   Leu Leu Asp Glu Pro Glu Asn Leu Asp Asp Glu Ala Lys Gly Ala Gly
   530 535 540
   Pro Glu Ile Glu His Leu Glu Phe Lys Ile Gly Val Glu Pro Lys Asp
   545 550 555 560
   Ser Leu Pro Phe Thr Val Ile Leu Val Ala Ser Thr Arg Gln Glu Lys
   565 570 575
   Ala Ala Trp Thr Ser Asp Ile Ile Gln Cys Val Asp Asn Ile Arg Cys
   580 585 590
   Asn Gly Leu Met Met Asn Ala Phe Glu Glu Asn Ser Lys Val Thr Val
   595 600 605
   Pro Gln Met Ile Lys Ser Asp Ala Ser Leu Tyr Cys Asp Asp Val Asp
   610 615 620
   Ile Arg Phe Ser Lys Thr Met Asn Ser Cys Lys Val Leu Gln Ile Arg
   625 630 635 640
   Tyr Ala Ser Val Glu Arg Leu Leu Glu Arg Leu Thr Asp Leu Arg Phe
   645 650 655
   Leu Ser Ile Asp Phe Leu Asn Thr Phe Leu His Ser Tyr Arg Val Phe
   660 665 670
   Thr Asn Ala Met Val Val Leu Asp Lys Leu Ile Asn Ile Tyr Arg Lys
   675 680 685
   Pro Met Ser Ala Ile Pro Ala Arg Ser Leu Glu Leu Leu Phe Ser Ser
   690 695 700
   Ser His Asn Ala Lys Leu Leu Tyr Gly Asp Ala Pro Lys Ser Pro Arg
   705 710 715 720
   Ala Ser Arg Lys Phe Ser Ser Pro Pro Pro Leu Ala Ile Gly Thr Ser
   725 730 735
   Ser Pro Ser Arg Arg Arg Lys Leu Ser Leu Asn Ile Pro Ile Ile Thr
   740 745 750
   Gly Gly Lys Ala Leu Glu Leu Ala Ser Leu Gly Cys Ser Ser Asp Ser
   755 760 765
   Tyr Ala Asn Ile His Ser Pro Ile Ser Pro Phe Gly Lys Thr Thr Leu
   770 775 780
   Asp Thr Gly Lys Leu Cys Met Ala Ser Ser Leu Pro Lys Thr Pro Glu
   785 790 795 800
   Glu Ile Asp Val Pro Ala Thr Ile Pro Glu Lys Pro Gly Glu Leu Ser
   805 810 815
   Ala Ser Arg Lys His Ser Ser Asp Val Leu Lys Glu Glu Ser Glu Asp
   820 825 830
   Asp Gln Asn His Ser Asp Glu Asp Asn Thr Glu Val Ser Pro Val Lys
   835 840 845
   Ser Pro Pro Thr Pro Lys Ser Phe Leu Asn Arg Thr Ile Thr Glu Phe
   850 855 860
   Pro Phe Phe Asn Tyr Asn Asn Gly Ile Leu Met Thr Thr Cys Arg Asp
   865 870 875 880
   Leu Val Asp Asn Asn Arg Ser Thr Leu Ser Ala Thr Ser Ala Phe Ala
   885 890 895
   Ile Ala Thr Ala Gly Ala Asn Glu Gly Pro Ser Asn Lys Glu Val Phe
   900 905 910
   Arg Arg Met Ser Leu Ala Asn Thr Gly Phe Ser Ser Asp Gln Arg Asn
   915 920 925
   Ile Asp Lys Glu Phe Val Ile Arg Arg Ala Ala Thr Asn Arg Val Leu
   930 935 940
   Asn Val Leu Arg His Trp Val Thr Lys His Thr Gln Asp Phe Asp Thr
   945 950 955 960
   Asp Asp Thr Leu Lys Tyr Arg Val Ile Cys Phe Leu Glu Glu Val Met
   965 970 975
   His Asp Pro Asp Leu Leu Thr Gln Glu Arg Lys Ala Ala Ala Asn Ile
   980 985 990
   Ile Arg Thr Leu Thr Leu Glu Glu Thr Thr Glu Gln His Ser Met Leu
   995 1000 1005
   Glu Glu Val Ile Leu Met Thr Glu Gly Val Lys Thr Glu Pro Phe Glu
   1010 1015 1020
   Asn His Pro Ala Leu Glu Ile Ala Glu Gln Leu Thr Leu Leu Asp His
   1025 1030 1035 1040
   Leu Val Phe Lys Ser Ile Pro Tyr Glu Glu Phe Phe Gly Gln Gly Trp
   1045 1050 1055
   Met Lys Ala Glu Lys Tyr Glu Arg Thr Pro Tyr Ile Met Lys Thr Thr
   1060 1065 1070
   Lys His Phe Asn His Val Ser Asn Phe Ile Ala Ser Glu Ile Ile Arg
   1075 1080 1085
   Asn Glu Asp Ile Ser Ala Arg Ala Ser Ala Ile Glu Lys Trp Val Ala
   1090 1095 1100
   Val Ala Asp Ile Cys Arg Cys Leu His Asn Tyr Asn Ala Val Leu Glu
   1105 1110 1115 1120
   Ile Thr Ser Ser Ile Asn Arg Ser Ala Ile Phe Arg Leu Lys Lys Thr
   1125 1130 1135
   Trp Leu Lys Val Ser Lys Gln Thr Lys Ser Leu Leu Asp Lys Leu Gln
   1140 1145 1150
   Lys Leu Val Ser Ser Asp Gly Arg Phe Lys Asn Leu Arg Glu Ser Leu
   1155 1160 1165
   Arg Asn Cys Asp Pro Pro Cys Val Pro Tyr Leu Gly Met Tyr Leu Thr
   1170 1175 1180
   Asp Leu Val Phe Ile Glu Glu Gly Thr Pro Asn Tyr Thr Glu Asp Gly
   1185 1190 1195 1200
   Leu Val Asn Phe Ser Lys Met Arg Met Ile Ser His Ile Ile Arg Glu
   1205 1210 1215
   Ile Arg Gln Phe Gln Gln Thr Thr Tyr Lys Ile Asp Pro Gln Pro Lys
   1220 1225 1230
   Val Ile Gln Tyr Leu Leu Asp Glu Ser Phe Met Leu Asp Glu Glu Ser
   1235 1240 1245
   Leu Tyr Glu Ser Ser Leu Leu Ile Glu Pro Lys Leu Pro Thr
   1250 1255 1260
<210> 3
   <211> 1244
   <212> PRT
   <213> Rattus norvegicus
<400> 3
   Met Gln Lys Ala Ile Arg Leu Asn Asp Gly His Val Val Ser Leu Gly
   1 5 10 15
   Leu Leu Ala Gln Arg Asp Gly Thr Arg Lys Gly Tyr Leu Ser Lys Arg
   20 25 30
   Ser Ser Asp Asn Pro Lys Trp Gln Thr Lys Trp Phe Ala Leu Leu Gln
   35 40 45
   Asn Leu Leu Phe Tyr Phe Glu Ser Asp Ser Ser Ser Arg Pro Ser Gly
   50 55 60
   Leu Tyr Leu Leu Glu Gly Ser Ile Cys Lys Arg Met Pro Ser Pro Lys
   65 70 75 80
   Arg Gly Thr Ser Ser Lys Glu Ser Asp Lys Gln His His Tyr Phe Thr
   85 90 95
   Val Asn Phe Ser Asn Asp Ser Gln Lys Ser Leu Glu Leu Arg Thr Asp
   100 105 110
   Asp Ser Lys Asp Cys Asp Glu Trp Val Ala Ala Ile Ala Arg Ala Ser
   115 120 125
   Tyr Lys Ile Leu Ala Thr Glu His Glu Ala Leu Met Gln Lys Tyr Leu
   130 135 140
   His Leu Leu Gln Val Val Glu Thr Glu Lys Thr Val Ala Lys Gln Leu
   145 150 155 160
   Arg Gln Gln Leu Glu Asp Gly Glu Val Glu Ile Glu Arg Leu Lys Ala
   165 170 175
   Glu Ile Ala Asn Leu Ile Lys Asp Asn Glu Arg Ile Gln Ser Asn Gln
   180 185 190
   Leu Val Ala Pro Glu Asp Glu Asp Ser Asp Ile Lys Lys Ile Lys Lys
   195 200 205
   Val Gln Ser Phe Leu Arg Gly Trp Leu Cys Arg Arg Lys Trp Lys Asn
   210 215 220
   Ile Ile Gln Asp Tyr Ile Arg Ser Pro His Ala Asp Ser Met Arg Lys
   225 230 235 240
   Arg Asn Gln Val Val Phe Ser Met Leu Glu Ala Glu Ala Glu Tyr Val
   245 250 255
   Gln Gln Leu His Ile Leu Val Asn Asn Phe Leu Arg Pro Leu Arg Met
   260 265 270
   Ala Ala Ser Ser Lys Lys Pro Pro Ile Thr His Asp Asp Val Ser Ser
   275 280 285
   Ile Phe Leu Asn Ser Glu Thr Ile Met Phe Leu His Gln Ile Phe Tyr
   290 295 300
   Gln Gly Leu Lys Ala Arg Ile Ala Ser Trp Pro Thr Leu Val Leu Ala
   305 310 315 320
   Asp Leu Phe Asp Ile Leu Leu Pro Met Leu Asn Ile Tyr Gln Glu Phe
   325 330 335
   Val Arg Asn His Gln Tyr Ser Leu Gln Ile Leu Ala His Cys Lys Gln
   340 345 350
   Asn Arg Asp Phe Asp Lys Leu Leu Lys Gln Tyr Glu Ala Lys Pro Asp
   355 360 365
   Cys Glu Glu Arg Thr Leu Glu Thr Phe Leu Thr Tyr Pro Met Phe Gln
   370 375 380
   Ile Pro Arg Tyr Ile Leu Thr Leu His Glu Leu Leu Ala His Thr Pro
   385 390 395 400
   His Glu His Val Glu Arg Asn Ser Leu Asp Tyr Ala Lys Ser Lys Leu
   405 410 415
   Glu Glu Leu Ser Arg Val Met His Asp Glu Val Ser Glu Thr Glu Asn
   420 425 430
   Ile Arg Lys Asn Leu Ala Ile Glu Arg Met Ile Thr Glu Gly Cys Glu
   435 440 445
   Ile Leu Leu Asp Thr Ser Gln Thr Phe Val Arg Gln Gly Ser Leu Ile
   450 455 460
   Gln Val Pro Met Ser Glu Lys Gly Lys Ile Asn Lys Gly Arg Leu Gly
   465 470 475 480
   Ser Leu Ser Leu Lys Lys Glu Gly Glu Arg Gln Cys Phe Leu Phe Ser
   485 490 495
   Lys His Leu Ile Ile Cys Thr Arg Gly Ser Gly Ser Lys Leu His Leu
   500 505 510
   Thr Lys Asn Gly Val Ile Ser Leu Ile Asp Cys Thr Leu Leu Asp Asp
   515 520 525
   Pro Glu Asn Met Asp Asp Asp Gly Lys Gly Gln Glu Val Asp His Leu
   530 535 540
   Asp Phe Lys Ile Trp Val Glu Pro Lys Asp Ser Pro Pro Phe Thr Val
   545 550 555 560
   Ile Leu Val Ala Ser Ser Arg Gln Glu Lys Ala Ala Trp Thr Ser Asp
   565 570 575
   Ile Ile Gln Cys Val Asp Asn Ile Arg Cys Asn Gly Leu Met Met Asn
   580 585 590
   Ala Phe Glu Glu Asn Ser Lys Val Thr Val Pro Gln Met Ile Lys Ser
   595 600 605
   Asp Ala Ser Leu Tyr Cys Asp Asp Val Asp Ile Arg Phe Ser Lys Thr
   610 615 620
   Met Asn Ser Cys Lys Val Leu Gln Ile Arg Tyr Ala Ser Val Glu Arg
   625 630 635 640
   Leu Leu Glu Arg Leu Thr Asp Leu Arg Phe Leu Ser Ile Asp Phe Leu
   645 650 655
   Asn Thr Phe Leu His Ser Tyr Arg Val Phe Thr Asp Ala Val Val Val
   660 665 670
   Leu Asp Lys Leu Ile Ser Ile Tyr Lys Lys Pro Ile Thr Ala Ile Pro
   675 680 685
   Ala Arg Ser Leu Glu Leu Leu Phe Ser Ser Ser His Asn Thr Lys Leu
   690 695 700
   Leu Tyr Gly Asp Ala Pro Lys Ser Pro Arg Ala Ser Arg Lys Phe Ser
   705 710 715 720
   Ser Pro Pro Pro Leu Ala Ile Gly Thr Ser Ser Pro Val Arg Arg Arg
   725 730 735
   Lys Leu Ser Leu Asn Ile Pro Ile Ile Thr Gly Gly Lys Ala Leu Glu
   740 745 750
   Leu Ala Ser Leu Gly Cys Pro Ser Asp Gly Tyr Thr Asn Ile His Ser
   755 760 765
   Pro Ile Ser Pro Phe Gly Lys Thr Thr Leu Asp Thr Ser Lys Leu Cys
   770 775 780
   Val Ala Ser Ser Leu Thr Arg Thr Pro Glu Glu Ile Asp Met Thr Thr
   785 790 795 800
   Leu Glu Glu Ser Ser Gly Phe Arg Lys Pro Thr Ser Asp Ile Leu Lys
   805 810 815
   Glu Glu Ser Asp Asp Asp Gln Ser Asp Val Asp Asp Thr Glu Val Ser
   820 825 830
   Pro Pro Thr Pro Lys Ser Phe Arg Asn Arg Ile Thr Gln Glu Phe Pro
   835 840 845
   Leu Phe Asn Tyr Asn Ser Gly Ile Met Met Thr Cys Arg Asp Leu Met
   850 855 860
   Asp Ser Asn Arg Ser Pro Leu Ser Ala Thr Ser Ala Phe Ala Ile Ala
   865 870 875 880
   Thr Ala Gly Ala Asn Glu Ser Pro Ala Asn Lys Glu Ile Tyr Arg Arg
   885 890 895
   Met Ser Leu Ala Asn Thr Gly Tyr Ser Ser Asp Gln Arg Asn Ile Asp
   900 905 910
   Lys Glu Phe Val Ile Arg Arg Ala Ala Thr Asn Arg Val Leu Asn Val
   915 920 925
   Leu Arg His Trp Val Thr Lys His Ser Gln Asp Phe Glu Thr Asp Asp
   930 935 940
   Leu Leu Lys Tyr Lys Val Ile Cys Phe Leu Glu Glu Val Met His Asp
   945 950 955 960
   Pro Asp Leu Leu Pro Gln Glu Arg Lys Ala Ala Ala Asn Ile Met Arg
   965 970 975
   Thr Leu Thr Gln Glu Glu Ile Thr Glu Asn His Ser Met Leu Asp Glu
   980 985 990
   Leu Leu Leu Met Thr Glu Gly Val Lys Thr Glu Pro Phe Glu Asn His
   995 1000 1005
   Ser Ala Met Glu Ile Ala Glu Gln Leu Thr Leu Leu Asp His Leu Val
   1010 1015 1020
   Phe Lys Ser Ile Pro Tyr Glu Glu Phe Phe Gly Gln Gly Trp Met Lys
   1025 1030 1035 1040
   Ala Asp Lys Asn Glu Arg Thr Pro Tyr Ile Met Lys Thr Thr Arg His
   1045 1050 1055
   Phe Asn His Ile Ser Asn Leu Ile Ala Ser Glu Ile Leu Arg Asn Glu
   1060 1065 1070
   Glu Val Ser Ala Arg Ala Ser Thr Ile Glu Lys Trp Val Ala Val Ala
   1075 1080 1085
   Asp Ile Cys Arg Cys Leu His Asn Tyr Asn Ala Val Leu Glu Ile Thr
   1090 1095 1100
   Ser Ser Ile Asn Arg Ser Ala Ile Phe Arg Leu Lys Lys Thr Trp Leu
   1105 1110 1115 1120
   Lys Val Ser Lys Gln Thr Lys Ser Leu Phe Asp Lys Leu Gln Lys Leu
   1125 1130 1135
   Val Ser Ser Asp Gly Arg Phe Lys Asn Leu Arg Glu Thr Leu Arg Asn
   1140 1145 1150
   Cys Asp Pro Pro Cys Val Pro Tyr Leu Gly Met Tyr Leu Thr Asp Leu
   1155 1160 1165
   Ala Phe Leu Glu Glu Gly Thr Pro Asn Tyr Thr Glu Asp Gly Leu Val
   1170 1175 1180
   Asn Phe Ser Lys Met Arg Met Ile Ser His Ile Ile Arg Glu Ile Arg
   1185 1190 1195 1200
   Gln Phe Gln Gln Thr Thr Tyr Lys Ile Glu Pro Gln Pro Lys Val Thr
   1205 1210 1215
   Gln Tyr Leu Val Asp Glu Thr Phe Val Leu Asp Asp Glu Ser Leu Tyr
   1220 1225 1230
   Glu Ala Ser Leu Arg Ile Glu Pro Lys Leu Pro Thr
   1235 1240